# EUROPEAN PATENT APPLICATION

(11) **EP 4 011 909 A1**
(43) Date of publication of application: **15.06.2022**
(21) Application number: 20849609.1
(22) Date of filing: 31.07.2020
(51) Int. Cl.: C07K 14/725, C12N 15/12, C12N 15/63, C12N 15/10, A61K 38/17, A61P 35/00

(54) **HIGH AFFINITY T-CELL RECEPTOR CAPABLE OF IDENTIFYING NY-ESO-1 ANTIGEN**

(30) Priority: 02.08.2019 CN 201910713184
(71) Applicant: XLifeSc, Ltd., Zhuhai, Guangdong 519031 (CN)
(72) Inventor: LI, Yi, Guangzhou, Guangdong 510663 (CN); CHEN, Liang, Guangzhou, Guangdong 510663 (CN); LI, Xiaolin, Guangzhou, Guangdong 510663 (CN)
(74) Representative: Grünecker Patent- und Rechtsanwälte PartG mbB
(86) International application number: PCT/CN2020/106291
(87) International publication number: WO 2021/023116

(57) **Abstract**

Provided is a T-cell receptor (TCR) having the characteristic of binding a SLLMWITQC-HLA A0201 complex. The binding affinity to the SLLMWITQC-HLA A0201 complex is at least 2 times that of a wild-type TCR to the SLLMWITQC-HLA A0201 complex. The TCR may be used alone or in combination with a therapeutic agent, so as to target a tumor cell presenting the SLLMWITQC-HLA A0201 complex.

## Description

### TECHNICAL FIELD

The present disclosure relates to the field of biotechnology and, in particular, to a T-cell receptor (TCR) capable of recognizing a polypeptide derived from NY-ESO-1 protein. The present disclosure further relates to the preparation and uses of such receptors.

### BACKGROUND

There are only two types of molecules that can recognize antigens in a specific manner. One is immunoglobulin or antibody and the other is T-cell receptor (TCR), which is α/β or γ/δ heterodimeric glycoprotein on cell membrane. The physical repertoire of TCR of an immune system is generated in thymus through V(D)J recombination, followed by positive and negative selections. In the peripheral environment, TCRs mediate the recognition of specific major histocompatibility complex-peptide complexes (pMHC) by T cells and, as such, are essential to the immunological functioning of cells in the immune system.

TCR is the only receptor for presenting specific peptide antigens in a major histocompatibility complex (MHC). The exogenous or endogenous peptides may be the only sign of abnormality in a cell. In the immune system, once antigen-specific TCRs bind with pMHC complexes, it causes direct physical contact of a T cell and an antigen-presenting cell (APC). Then, the interaction of other membrane molecules in the T cell and APC occurs and the subsequent cell signaling and other physiological responses are initiated so that a range of different antigen-specific T cells exert immune effects on their target cells.

Molecular ligands of MHC class I and class II corresponding to TCRs are also proteins of the immunoglobulin superfamily but are specific for antigen presentation, and different individuals have different MHCs, thereby presenting different short peptides in one protein antigen to the surface of respective APC cells. Human MHC is commonly referred to as HLA gene or HLA complex.

The short peptide SLLMWITQC is derived from the NY-ESO-1 protein expressed by various tumor cells (Chen et al., (1997) PNAS USA 94 1914-1918). Type I HLA molecules of tumor cells present short peptides derived from NY-ESO-1, including SLLMWITQC. Therefore, SLLMWITQC-HLA A0201 complex provides a marker for TCR targeting tumor cells. The TCR capable of binding to SLLMWITQC-HLA A0201 complex has high application value for treating tumors. For example, a TCR capable of targeting the tumor cell marker can be used to deliver a cytotoxic agent or immunostimulatory agent to target cells, or have the same transformed into T cells, such that the T cell expressing the TCR can destroy the tumor cells and can be administered to a patient during the course of treatment of so called adoptive immunotherapy. For the former purpose, the ideal TCR has higher affinity, allowing the TCR to reside on the targeted cells for a long period of time. For the latter purpose, it is preferred to use a TCR with medium affinity. Accordingly, those skilled in the art are directed to developing TCRs that can be used to target tumor cell markers for different purposes.

### SUMMARY

An object of the present disclosure is to provide a TCR with high affinity for SLLMWITQC-HLA A0201 complex.

Another object of the present disclosure is to provide a method for preparing the TCR of the preceding type and a use of the TCR of the preceding type.

In a first aspect of the present disclosure, a T-cell receptor (TCR) is provided, which has an activity of binding to SLLMWITQC-HLA A0201 complex.

In another preferred embodiment, the TCR has an activity of binding to SLLMWITQC-HLA A0201 complex and comprises a TCR α chain variable domain and a TCR β chain variable domain; wherein the TCR contains a mutation in the α chain variable domain shown in SEQ ID NO: 1, and the mutation is selected from one or more of T27G/Q/V, S28W/T/N, I29A/P, N30Q, S51T, N52G, E53Q, R54A, A90G/L/M, T91F/W/Y, A93E/H/M/Q/R/S, N94A/D/F/H/S/W, G95A, K96R/Q/S/L/T/W, I97W/V/M/P and I98F/E/D/H/L/Q/R/T/Y, wherein amino acid residues are numbered as shown in SEQ ID NO: 1; and/or the TCR contains a mutation in the β chain variable domain shown in SEQ ID NO: 2, and the mutation is selected from one or more of N51H, N52G, V53A, P54L, A93S, S95Q, L96R/K/H/Q, G97A, S98A/G/P, N99G, E100P, Q101W and Y102I, wherein amino acid residues are numbered as shown in SEQ ID NO: 2.

In another preferred embodiment, the TCR comprises a TCR α chain variable domain and a TCR β chain variable domain, the TCR α chain variable domain comprises CDR1α, CDR2α and CDR3α, and the TCR β chain variable domain comprises CDR1β, CDR2β and CDR3β, wherein the amino acid sequence of CDR1β is SGHDY.

Preferably, the amino acid sequence of CDR2α is IRSNERE.

In another preferred embodiment, the amino acid sequence of CDR1α is selected from TSINN, QTPQN, VNPQN and GSIQN.

In another preferred embodiment, the amino acid sequence of CDR2β is selected from FNNNVP and FNHGAL.

In another preferred embodiment, the amino acid sequence of CDR3β is selected from ASQLGSNEQY and ASQLGPNEQY

In a preferred embodiment of the present disclosure, the affinity of the TCR for SLLMWITQC-HLA A0201 complex is at least twice, preferably at least five times, more preferably at least ten times that of a wild type TCR.

In another preferred embodiment, the affinity of the TCR for SLLMWITQC-HLA A0201 complex is at least 50 times, preferably at least 100 times, more preferably at least 500 times, most preferably at least 1000 times that of a wild type TCR.

In another preferred embodiment, the affinity of the TCR for SLLMWITQC-HLA A0201 complex is at least 10⁴ times, preferably at least 10⁵ times, more preferably at least 10⁶ times that of a wild type TCR.

Specifically, the dissociation equilibrium constant K_{D} of the TCR to SLLMWITQC-HLA A0201 complex is K_{D} ≤ 20 µM.

In another preferred embodiment, the dissociation equilibrium constant K_{D} of the TCR to SLLMWITQC-HLA A0201 complex is 5 µM ≤ K_{D} ≤ 10 µM, preferably 0.1 µM ≤ K_{D} ≤ 1 µM, more preferably 1 nM ≤ K_{D} ≤ 100 nM.

In another preferred embodiment, the dissociation equilibrium constant K_{D} of the TCR to SLLMWITQC-HLA A0201 complex is 100 pM ≤ K_{D} ≤ 1000 pM, preferably 10 pM ≤ K_{D} ≤ 100 pM.

In another preferred embodiment, the TCR has CDRs selected from the group consisting of:

| CDR No. | CDR1α | CDR2α | CDR3α | CDR1β | CDR2β | CDR3β |
|---|---|---|---|---|---|---|
| 27 | TSINN | IRSNERE | AFDQNGKII | SGHDY | FNNNVP | ASSLGSNEQY |
| 22 | TSINN | IRSNERE | MFDRNGKII | SGHDY | FNNNVP | ASSLGSNEQY |
| 1 | TSINN | IRSNERE | ATDANGSIF | SGHDY | FNNNVP | ASSLGSNEQY |
| 2 | TSINN | IRSNERE | ATDANGRWR | SGHDY | FNNNVP | ASSLGSNEQY |
| 3 | TSINN | IRSNERE | ATDANGWVQ | SGHDY | FNNNVP | ASSLGSNEQY |
| 4 | TSINN | IRSNERE | ATDANGWMQ | SGHDY | FNNNVP | ASSLGSNEQY |
| 5 | TSINN | IRSNERE | ATDANGLPH | SGHDY | FNNNVP | ASSLGSNEQY |
| 6 | TSINN | IRSNERE | ATDANGTIE | SGHDY | FNNNVP | ASSLGSNEQY |
| 7 | TSINN | IRSNERE | ATDANGLPQ | SGHDY | FNNNVP | ASSLGSNEQY |
| 8 | TSINN | IRSNERE | ATDANGLPE | SGHDY | FNNNVP | ASSLGSNEQY |
| 9 | TSINN | IRSNERE | ATDANGSMQ | SGHDY | FNNNVP | ASSLGSNEQY |
| 10 | TSINN | IRSNERE | ATDANGLPT | SGHDY | FNNNVP | ASSLGSNEQY |
| 11 | TSINN | IRSNERE | ATDAQGRWI | SGHDY | FNNNVP | ASSLGSNEQY |
| 12 | TSINN | IRSNERE | ATDANGSID | SGHDY | FNNNVP | ASSLGSNEQY |
| 13 | TSINN | IRSNERE | AYDQNGKII | SGHDY | FNNNVP | ASSLGSNEQY |
| 14 | TSINN | IRSNERE | ATDANGQPR | SGHDY | FNNNVP | ASSLGSNEQY |
| 15 | TSINN | IRSNERE | LYDQNGKII | SGHDY | FNNNVP | ASSLGSNEQY |
| 16 | TSINN | IRSNERE | ATDANAQVR | SGHDY | FNNNVP | ASSLGSNEQY |
| 17 | TSINN | IRSNERE | ATDANGRPH | SGHDY | FNNNVP | ASSLGSNEQY |
| 18 | TSINN | IRSNERE | ATDANGRMY | SGHDY | FNNNVP | ASSLGSNEQY |
| 19 | TSINN | IRSNERE | GYDENGKII | SGHDY | FNNNVP | ASSLGSNEQY |
| 20 | TSINN | IRSNERE | ATDANGLIQ | SGHDY | FNNNVP | ASSLGSNEQY |
| 21 | TSINN | IRSNERE | ATDANGRML | SGHDY | FNNNVP | ASSLGSNEQY |
| 23 | TSINN | IRSNERE | MFDQNGKII | SGHDY | FNNNVP | ASSLGSNEQY |
| 24 | TSINN | IRSNERE | AYDHSGKII | SGHDY | FNNNVP | ASSLGSNEQY |
| 25 | TSINN | IRSNERE | AYDQDGKII | SGHDY | FNNNVP | ASSLGSNEQY |
| 26 | TSINN | IRSNERE | AYDEAGKII | SGHDY | FNNNVP | ASSLGSNEQY |
| 28 | TSINN | IRSNERE | AYDMHGKII | SGHDY | FNNNVP | ASSLGSNEQY |
| 29 | TSINN | IRSNERE | AYDQWGKII | SGHDY | FNNNVP | ASSLGSNEQY |
| 30 | TSINN | IRSNERE | AWDSWGKII | SGHDY | FNNNVP | ASSLGSNEQY |
| 31 | TSINN | IRSNERE | ATDAWGLPI | SGHDY | FNNNVP | ASSLGSNEQY |
| 32 | TSINN | IRSNERE | ATDAFGSVI | SGHDY | FNNNVP | ASSLGSNEQY |
| 33 | TSINN | IRSNERE | ATDANGTVL | SGHDY | FNNNVP | ASSLGSNEQY |
| 34 | TSINN | IRSNERE | ATDANGTVR | SGHDY | FNNNVP | ASSLGSNEQY |
| 35 | TSINN | IRSNERE | ATDANGKII | SGHDY | FNNNVP | SSQLGSNEQY |
| 36 | TSINN | IRSNERE | ATDANGKII | SGHDY | FNNNVP | ASQHGSNEQY |
| 37 | TSINN | IRSNERE | ATDANGKII | SGHDY | FNNNVP | ASQQGSNEQY |
| 38 | TSINN | IRSNERE | ATDANGKII | SGHDY | FNNNVP | ASQQGANEQY |
| 39 | TSINN | IRSNERE | ATDANGKII | SGHDY | FNNNVP | ASQQAANEQY |
| 40 | TSINN | IRSNERE | ATDANGKII | SGHDY | FNNNVP | ASQKGSNEQY |
| 41 | TSINN | IRSNERE | ATDANGKII | SGHDY | FNNNVP | ASSLGSGPWI |
| 42 | TSINN | IRSNERE | ATDANGKII | SGHDY | FNNNVP | ASQQGGNEQY |
| 43 | TSINN | IRSNERE | ATDANGKII | SGHDY | FNNNVP | ASQLAGNEQY |
| 44 | TSINN | IRTGQAE | ATDANGKII | SGHDY | FNNNVP | ASSLGSNEQY |
| 45 | TSINN | IRSNERE | ATDANGRWI | SGHDY | FNNNVP | ASSLGSNEQY |
| 46 | TSINN | IRSNERE | ATDANGKII | SGHDY | FNHGAL | ASSLGSNEQY |
| 47 | TSINN | IRSNERE | ATDANGKII | SGHDY | FNNNVP | ASQRGSNEQY |
| 48 | TSINN | IRSNERE | AWDSWGKII | SGHDY | FNHGAL | ASSLGSNEQY |
| 49 | TSINN | IRSNERE | AYDQWGKII | SGHDY | FNHGAL | ASSLGSNEQY |
| 50 | TSINN | IRSNERE | AWDSWGKII | SGHDY | FNNNVP | ASQLGSNEQY |
| 51 | TSINN | IRSNERE | AWDSWGKII | SGHDY | FNNNVP | ASQLGPNEQY |
| 52 | GWAQN | IRSNERE | ATDANGKII | SGHDY | FNNNVP | ASSLGSNEQY |
| 53 | GSIQN | IRSNERE | ATDANGKII | SGHDY | FNNNVP | ASSLGSNEQY |
| 54 | GSIQN | IRSNERE | AYDQWGKII | SGHDY | FNHGAL | ASSLGSNEQY |
| 55 | GSIQN | IRSNERE | AWDSWGKII | SGHDY | FNHGAL | ASSLGSNEQY |
| 56 | GSIQN | IRSNERE | ATDAFGSVI | SGHDY | FNHGAL | ASSLGSNEQY |
| 57 | GSIQN | IRSNERE | ATDANGTVQ | SGHDY | FNHGAL | ASSLGSNEQY |
| 58 | GSIQN | IRSNERE | AYDQWGKII | SGHDY | FNHGAL | ASQLGSNEQY |
| 59 | GSIQN | IRSNERE | AWDSWGKII | SGHDY | FNHGAL | ASQLGSNEQY |
| 60 | GSIQN | IRSNERE | ATDAFGSVI | SGHDY | FNHGAL | ASQLGSNEQY |
| 61 | GSIQN | IRSNERE | ATDANGTVQ | SGHDY | FNHGAL | ASQLGSNEQY |
| 62 | GSIQN | IRSNERE | AYDQWGKII | SGHDY | FNHGAL | ASQLGPNEQY |
| 63 | GSIQN | IRSNERE | AWDSWGKII | SGHDY | FNHGAL | ASQLGPNEQY |
| 64 | GSIQN | IRSNERE | ATDAFGSVI | SGHDY | FNHGAL | ASQLGPNEQY |
| 65 | GSIQN | IRSNERE | ATDANGTVQ | SGHDY | FNHGAL | ASQLGPNEQY |
| 66 | QTPQN | IRSNERE | ATDANGKII | SGHDY | FNNNVP | ASSLGSNEQY |
| 67 | QTPQN | IRSNERE | ATDANGKII | SGHDY | FNHGAL | ASSLGSNEQY |
| 68 | QTPQN | IRSNERE | ATDANGKII | SGHDY | FNNNVP | ASQLGSNEQY |
| 69 | QTPQN | IRSNERE | ATDANGKII | SGHDY | FNNNVP | ASQLGPNEQY |
| 70 | QTPQN | IRSNERE | AYDQWGKII | SGHDY | FNHGAL | ASSLGSNEQY |
| 71 | QTPQN | IRSNERE | AWDSWGKII | SGHDY | FNHGAL | ASSLGSNEQY |
| 72 | QTPQN | IRSNERE | ATDAFGSVI | SGHDY | FNHGAL | ASSLGSNEQY |
| 73 | QTPQN | IRSNERE | ATDANGTVQ | SGHDY | FNHGAL | ASSLGSNEQY |
| 74 | QTPQN | IRSNERE | AYDQWGKII | SGHDY | FNHGAL | ASQLGSNEQY |
| 75 | QTPQN | IRSNERE | AWDSWGKII | SGHDY | FNHGAL | ASQLGSNEQY |
| 76 | QTPQN | IRSNERE | ATDAFGSVI | SGHDY | FNHGAL | ASQLGSNEQY |
| 77 | QTPQN | IRSNERE | ATDANGTVQ | SGHDY | FNHGAL | ASQLGSNEQY |
| 78 | QTPQN | IRSNERE | AYDQWGKII | SGHDY | FNHGAL | ASQLGPNEQY |
| 79 | QTPQN | IRSNERE | AWDSWGKII | SGHDY | FNHGAL | ASQLGPNEQY |
| 80 | QTPQN | IRSNERE | ATDAFGSVI | SGHDY | FNHGAL | ASQLGPNEQY |
| 81 | QTPQN | IRSNERE | ATDANGTVQ | SGHDY | FNHGAL | ASQLGPNEQY |
| 82 | VNPQN | IRSNERE | ATDANGKII | SGHDY | FNNNVP | ASSLGSNEQY |
| 83 | VNPQN | IRSNERE | ATDANGKII | SGHDY | FNHGAL | ASSLGSNEQY |
| 84 | VNPQN | IRSNERE | ATDANGKII | SGHDY | FNNNVP | ASQLGSNEQY |
| 85 | VNPQN | IRSNERE | ATDANGKII | SGHDY | FNNNVP | ASQLGPNEQY |
| 86 | VNPQN | IRSNERE | AYDQWGKII | SGHDY | FNHGAL | ASSLGSNEQY |
| 87 | VNPQN | IRSNERE | AWDSWGKII | SGHDY | FNHGAL | ASSLGSNEQY |
| 88 | VNPQN | IRSNERE | ATDAFGSVI | SGHDY | FNHGAL | ASSLGSNEQY |
| 89 | VNPQN | IRSNERE | ATDANGTVQ | SGHDY | FNHGAL | ASSLGSNEQY |
| 90 | VNPQN | IRSNERE | AYDQWGKII | SGHDY | FNHGAL | ASQLGSNEQY |
| 91 | VNPQN | IRSNERE | AWDSWGKII | SGHDY | FNHGAL | ASQLGSNEQY |
| 92 | VNPQN | IRSNERE | ATDAFGSVI | SGHDY | FNHGAL | ASQLGSNEQY |
| 93 | VNPQN | IRSNERE | ATDANGTVQ | SGHDY | FNHGAL | ASQLGSNEQY |
| 94 | VNPQN | IRSNERE | AYDQWGKII | SGHDY | FNHGAL | ASQLGPNEQY |
| 95 | VNPQN | IRSNERE | AWDSWGKII | SGHDY | FNHGAL | ASQLGPNEQY |
| 96 | VNPQN | IRSNERE | ATDAFGSVI | SGHDY | FNHGAL | ASQLGPNEQY |
| 97 | VNPQN | IRSNERE | ATDANGTVQ | SGHDY | FNHGAL | ASQLGPNEQY |

In another preferred embodiment, the TCR is an αβ heterodimeric TCR; preferably, the TCR has an α chain constant region sequence TRAC^{∗}01 and a β chain constant region sequence TRBC1^{∗}01 or TRBC2^{∗}01.

In another preferred embodiment, the TCR is soluble.

In another preferred embodiment, the TCR is an αβ heterodimeric TCR or a single chain TCR.

In another preferred embodiment, the TCR of the present disclosure is an αβ heterodimeric TCR, and the α chain variable domain of the TCR comprises an amino acid sequence having at least 85%, preferably at least 90%, more preferably at least 92%, most preferably at least 94% (for example, may be at least 88%, 89%, 90%, 91%, 92%, 93%, 94%, 95%, 96%, 97%, 98%, 99%) of sequence homology with the amino acid sequence as shown in SEQ ID NO: 1; and/or the β chain variable domain of the TCR comprises an amino acid sequence having at least 90%, preferably at least 92%, more preferably at least 94%, most preferably at least 97% (for example, may be at least 91%, 92%, 93%, 94%, 95%, 96%, 97%, 98%, 99%) of sequence homology with the amino acid sequence as shown in SEQ ID NO: 2.

In another preferred embodiment, the TCR comprises (i) all or part of a TCR α chain other than its transmembrane domain and (ii) all or part of a TCR β chain other than its transmembrane domain, wherein both of (i) and (ii) comprise a variable domain and at least part of a constant domain of the TCR chain.

In another preferred embodiment, the TCR is an αβ heterodimeric TCR, and an artificial interchain disulfide bond is contained between an α chain variable region and a β chain constant region of the TCR.

In another preferred embodiment, one or more groups of amino acids selected from the following are substituted by cysteine residues forming the artificial interchain disulfide bond between the α chain variable region and the β chain constant region of the TCR:
an amino acid at position 46 of TRAV and an amino acid at position 60 of TRBC1^{∗}01 or TRBC2^{∗}01 exon 1;
an amino acid at position 47 of TRAV and an amino acid at position 61 of TRBC1^{∗}01 or TRBC2^{∗}01 exon 1;
an amino acid at position 46 of TRAV and an amino acid at position 61 of TRBC1^{∗}01 or TRBC2^{∗}01 exon 1; or
an amino acid at position 47 of TRAV and an amino acid at position 60 of TRBC1^{∗}01 or TRBC2^{∗}01 exon 1.

In another preferred embodiment, the TCR containing an artificial interchain disulfide bond between an α chain variable region and a β chain constant region comprises an α chain variable domain, a β chain variable domain and all or part of a β chain constant domain other than the transmembrane domain and comprises no α chain constant domain, wherein the α chain variable domain and the β chain of the TCR form a heterodimer.

In another preferred embodiment, the TCR containing an artificial interchain disulfide bond between an α chain variable region and a β chain constant region comprises (i) all or part of a TCR α chain other than its transmembrane domain and (ii) all or part of a TCR β chain other than its transmembrane domain, wherein both of (i) and (ii) comprise a variable domain and at least part of a constant domain of the TCR chain.

In another preferred embodiment, the TCR is an αβ heterodimeric TCR and comprises (i) all or part of a TCR α chain other than its transmembrane domain and (ii) all or part of a TCR β chain other than its transmembrane domain, wherein both of (i) and (ii) comprise a variable domain and at least part of a constant domain of the TCR chain, and an artificial interchain disulfide bond is contained between an α chain constant region and a β chain constant region.

In another preferred embodiment, one or more groups of amino acids selected from the following are substituted by cysteine residues forming the artificial interchain disulfide bond between the α chain constant region and the β chain constant region of the TCR:
Thr48 of TRAC^{∗}01 exon 1 and Ser57 of TRBC1^{∗}01 or TRBC2^{∗}01 exon 1;
Thr45 of TRAC^{∗}01 exon 1 and Ser77 of TRBC1^{∗}01 or TRBC2^{∗}01 exon 1;
Tyr10 of TRAC^{∗}01 exon 1 and Ser17 of TRBC1^{∗}01 or TRBC2^{∗}01 exon 1;
Thr45 of TRAC^{∗}01 exon 1 and Asp59 of TRBC1^{∗}01 or TRBC2^{∗}01 exon 1;
Ser15 of TRAC^{∗}01 exon 1 and Glu15 of TRBC1^{∗}01 or TRBC2^{∗}01 exon 1;
Arg53 of TRAC^{∗}01 exon 1 and Ser54 of TRBC1^{∗}01 or TRBC2^{∗}01 exon 1;
Pro89 of TRAC^{∗}01 exon 1 and Ala19 of TRBC1^{∗}01 or TRBC2^{∗}01 exon 1; and
Tyr10 of TRAC^{∗}01 exon 1 and Glu20 of TRBC1^{∗}01 or TRBC2^{∗}01 exon 1.

In another preferred embodiment, the TCR is a single chain TCR.

In another preferred embodiment, the TCR is a single chain TCR consisting of an α chain variable domain and a β chain variable domain, wherein the α chain variable domain and the β chain variable domain are linked by a flexible short peptide sequence (linker).

In another preferred embodiment, a hydrophobic core of the α chain variable domain and/or the β chain variable domain of the TCR is mutated.

In another preferred embodiment, the TCR, in which the hydrophobic core is mutated, is a single chain TCR consisting of an α variable domain and a β variable domain, wherein the α variable domain and the β variable domain are linked by a flexible short peptide sequence (linker).

In another preferred embodiment, the TCR of the present disclosure is a single chain TCR, and the α chain variable domain of the TCR comprises an amino acid sequence having at least 85%, preferably at least 90%, more preferably at least 92%, most preferably at least 94% (for example, may be at least 88%, 89%, 90%, 91%, 92%, 93%, 94%, 95%, 96%, 97%, 98%, 99%) of sequence homology with the amino acid sequence as shown in SEQ ID NO: 3; and/or the β chain variable domain of the TCR comprises an amino acid sequence having at least 90%, preferably at least 92%, more preferably at least 94%, most preferably at least 97% (for example, may be at least 91%, 92%, 93%, 94%, 95%, 96%, 97%, 98%, 99%) of sequence homology with the amino acid sequence as shown in SEQ ID NO: 4.

In another preferred embodiment, the amino acid sequence of the α chain variable domain of the TCR is selected from the group consisting of: SEQ ID NOs: 9-42 and 58-86; and/or the amino acid sequence of the β chain variable domain of the TCR is selected from the group consisting of: SEQ ID NOs: 38-53 and 87-102.

In another preferred embodiment, the TCR is selected from the group consisting of:

| TCR No. | Sequence of a chain variable domain SEQ ID NO: | Sequence of β chain variable domain SEQ ID NO: |
|---|---|---|
| s-27 | 35 | 4 |
| s-22 | 30 | 4 |
| s-1 | 9 | 4 |
| s-2 | 10 | 4 |
| s-3 | 11 | 4 |
| s-4 | 12 | 4 |
| s-5 | 13 | 4 |
| s-6 | 14 | 4 |
| s-7 | 15 | 4 |
| s-8 | 16 | 4 |
| s-9 | 17 | 4 |
| s-10 | 18 | 4 |
| s-11 | 19 | 4 |
| s-12 | 20 | 4 |
| s-13 | 21 | 4 |
| s-14 | 22 | 4 |
| s-15 | 23 | 4 |
| s-16 | 24 | 4 |
| s-17 | 25 | 4 |
| s-18 | 26 | 4 |
| s-19 | 27 | 4 |
| s-20 | 28 | 4 |
| s-21 | 29 | 4 |
| s-23 | 31 | 4 |
| s-24 | 32 | 4 |
| s-25 | 33 | 4 |
| s-26 | 34 | 4 |
| s-28 | 36 | 4 |
| s-29 | 37 | 4 |
| s-30 | 38 | 4 |
| s-31 | 39 | 4 |
| s-32 | 40 | 4 |
| s-33 | 41 | 4 |
| s-34 | 42 | 4 |
| s-35 | 3 | 43 |
| s-36 | 3 | 44 |
| s-37 | 3 | 45 |
| s-38 | 3 | 46 |
| s-39 | 3 | 47 |
| s-40 | 3 | 48 |
| s-41 | 3 | 49 |
| s-42 | 3 | 50 |
| s-43 | 3 | 51 |

In another preferred embodiment, the TCR is selected from the group consisting of:

| TCR No. | Sequence of a chain variable domain SEQ ID NO: | Sequence of β chain variable domain SEQ ID NO: |
|---|---|---|
| 27 | 82 | 2 |
| 22 | 77 | 2 |
| 1 | 56 | 2 |
| 2 | 57 | 2 |
| 3 | 58 | 2 |
| 4 | 59 | 2 |
| 5 | 60 | 2 |
| 6 | 61 | 2 |
| 7 | 62 | 2 |
| 8 | 63 | 2 |
| 9 | 64 | 2 |
| 10 | 65 | 2 |
| 11 | 66 | 2 |
| 12 | 67 | 2 |
| 13 | 68 | 2 |
| 14 | 69 | 2 |
| 15 | 70 | 2 |
| 16 | 71 | 2 |
| 17 | 72 | 2 |
| 18 | 73 | 2 |
| 19 | 74 | 2 |
| 20 | 75 | 2 |
| 21 | 76 | 2 |
| 23 | 78 | 2 |
| 24 | 79 | 2 |
| 25 | 80 | 2 |
| 26 | 81 | 2 |
| 28 | 83 | 2 |
| 29 | 84 | 2 |
| 30 | 85 | 2 |
| 31 | 86 | 2 |
| 32 | 87 | 2 |
| 33 | 88 | 2 |
| 34 | 89 | 2 |
| 35 | 1 | 108 |
| 36 | 1 | 109 |
| 37 | 1 | 110 |
| 38 | 1 | 111 |
| 39 | 1 | 112 |
| 40 | 1 | 113 |
| 41 | 1 | 114 |
| 42 | 1 | 115 |
| 43 | 1 | 116 |
| 44 | 90 | 2 |
| 45 | 91 | 2 |
| 46 | 1 | 117 |
| 47 | 1 | 118 |
| 48 | 85 | 117 |
| 49 | 84 | 117 |
| 50 | 85 | 119 |
| 51 | 85 | 120 |
| 52 | 92 | 2 |
| 53 | 93 | 2 |
| 54 | 94 | 117 |
| 55 | 95 | 117 |
| 56 | 96 | 117 |
| 57 | 97 | 117 |
| 58 | 94 | 121 |
| 59 | 95 | 121 |
| 60 | 96 | 121 |
| 61 | 97 | 121 |
| 62 | 94 | 122 |
| 63 | 95 | 122 |
| 64 | 96 | 122 |
| 65 | 97 | 122 |
| 66 | 98 | 2 |
| 67 | 98 | 117 |
| 68 | 98 | 119 |
| 69 | 98 | 120 |
| 70 | 99 | 117 |
| 71 | 100 | 117 |
| 72 | 101 | 117 |
| 73 | 102 | 117 |
| 74 | 99 | 121 |
| 75 | 100 | 121 |
| 76 | 101 | 121 |
| 77 | 102 | 121 |
| 78 | 99 | 122 |
| 79 | 100 | 122 |
| 80 | 101 | 122 |
| 81 | 102 | 122 |
| 82 | 103 | 2 |
| 83 | 103 | 117 |
| 84 | 103 | 119 |
| 85 | 103 | 120 |
| 86 | 104 | 117 |
| 87 | 105 | 117 |
| 88 | 106 | 117 |
| 89 | 107 | 117 |
| 90 | 104 | 121 |
| 91 | 105 | 121 |
| 92 | 106 | 121 |
| 93 | 107 | 121 |
| 94 | 104 | 122 |
| 95 | 105 | 122 |
| 96 | 106 | 122 |
| 97 | 107 | 122 |

In another preferred embodiment, a conjugate binds to an α chain and/or a β chain of the TCR at C- or N-terminal.

In another preferred embodiment, the conjugate that binds to the TCR is a detectable label, a therapeutic agent, a PK modified moiety or a combination thereof.

In another preferred embodiment, the therapeutic agent that binds to the TCR is an anti-CD3 antibody linked to the α or β chain of the TCR at C- or N-terminal.

In a second aspect of the present disclosure, a multivalent TCR complex is provided, which comprises at least two TCR molecules, wherein at least one TCR molecule is the TCR in the first aspect of the present disclosure.

In a third aspect of the present disclosure, a nucleic acid molecule is provided, which comprises a nucleic acid sequence for encoding the TCR molecule in the first aspect of the present disclosure or the multivalent TCR complex in the second aspect of the present disclosure or a complementary sequence thereof.

In a fourth aspect of the present disclosure, a vector is provided, which contains the nucleic acid molecule in the third aspect of the present disclosure.

In a fifth aspect of the present disclosure, a host cell is provided, which contains the vector in the fourth aspect of the present disclosure or has the exogenous nucleic acid molecule in the third aspect of the present disclosure integrated into a chromosome of the host cell.

In a sixth aspect of the present disclosure, an isolated cell is provided, which expresses the TCR in the first aspect of the present disclosure.

In a seventh aspect of the present disclosure, a pharmaceutical composition is provided, which contains a pharmaceutically acceptable carrier and the TCR in the first aspect of the present disclosure or the TCR complex in the second aspect of the present disclosure or the cell in the sixth aspect of the present disclosure.

In an eighth aspect of the present disclosure, a method for treating a disease is provided, which comprises administering an appropriate amount of the TCR in the first aspect of the present disclosure or the TCR complex in the second aspect of the present disclosure or the cell in the sixth aspect of the present disclosure or the pharmaceutical composition in the seventh aspect of the present disclosure to a subject in need thereof. Preferably, the disease is NY-ESO-1 positive tumor.

In a ninth aspect of the present disclosure, a use of the TCR in the first aspect of the present disclosure or the TCR complex in the second aspect of the present disclosure or the cell in the sixth aspect of the present disclosure is provided for preparation of a medicament for treating a tumor. Preferably, the tumor is NY-ESO-1 positive tumor.

In a preferred embodiment, the present disclosure provides the TCR, the TCR complex or the cell of the present disclosure for use as a medicament for treating a tumor; preferably, the tumor is NY-ESO-1 positive tumor.

In a tenth aspect of the present disclosure, a method for preparing the T-cell receptor (TCR) in the first aspect of the present disclosure is provided, which comprises the steps of:
(i) culturing the host cell in the fifth aspect of the present disclosure to express the TCR in the first aspect of the present disclosure; and
(ii) isolating or purifying the TCR.

It is to be understood that within the scope of the present disclosure, the preceding technical features of the present disclosure and the technical features specifically described hereinafter (as in embodiments) may be combined with each other to constitute a new or preferred technical solution, which will not be repeated herein one by one.

### BRIEF DESCRIPTION OF DRAWINGS

FIGS. 1a and 1b show the amino acid sequences of α and β chain variable domains of a wild type TCR capable of specifically binding to SLLMWITQC-HLA A0201 complex, respectively.
FIGS. 2a and 2b show the amino acid sequences of an α variable domain and a β chain variable domain of a single chain template TCR constructed in the present disclosure, respectively.
FIGS. 3a and 3b show the DNA sequences of an α variable domain and a β chain variable domain of a single chain template TCR constructed in the present disclosure, respectively.
FIGS. 4a and 4b are the amino acid sequence and nucleotide sequence of a linking short peptide (linker) of a single chain template TCR constructed in the present disclosure, respectively.
FIGS. 5-1 to 5-34 show the amino acid sequences of an α chain variable domain of a single chain TCR with high affinity for SLLMWITQC-HLA A0201 complex, respectively, wherein the mutated residues are underlined.
FIGS. 6a to 6i show the amino acid sequences of a β chain variable domain of a single chain TCR with high affinity for SLLMWITQC-HLA A0201 complex, respectively, wherein the mutated residues are underlined.
FIGS. 7a and 7b show the amino acid sequence and DNA sequence of a single chain template TCR constructed in the present disclosure, respectively.
FIGS. 8a and 8b show the amino acid sequences of α and β chains of a soluble reference TCR in the present disclosure, respectively.
FIGS. 9-1 to 9-52 show the amino acid sequences of an α chain variable domain of a heterodimeric TCR with high affinity for SLLMWITQC-HLA A0201 complex, respectively, wherein the mutated residues are underlined.
FIGS. 10a to 10o show the amino acid sequences of a β chain variable domain of a heterodimeric TCR with high affinity for SLLMWITQC-HLA A0201 complex, respectively, wherein the mutated residues are underlined.
FIGS. 11a and 11b show the extracellular amino acid sequences of α and β chains of a wild type TCR capable of specifically binding to SLLMWITQC-HLA A0201 complex, respectively.
FIG. 12 is a binding curve of a soluble reference TCR to SLLMWITQC-HLA A0201 complex.
FIG. 13 shows results of the INF-γ activation experiment (with a cell line as target cells) of effector cells transfected with a high affinity TCR of the present disclosure.
FIG. 14 shows results of the INF-γ activation experiment (with T2 loaded with specific short peptides as target cells) of effector cells transfected with a high affinity TCR of the present disclosure.
FIG. 15 shows results of the IL-2 activation experiment of effector cells transfected with a high affinity TCR of the present disclosure.
FIGS. 16a to 16f show results of the IncuCyte killing experiment on target cells of effector cells transfected with a high affinity TCR of the present disclosure.
FIGS. 17a and 17b show results of the LDH killing experiment on target cells of effector cells transfected with a high affinity TCR of the present disclosure.
FIGS. 18a to 18j show results of the redirection experiment of a fusion protein on effector cells.
FIGS. 19a and 19b show the amino acid sequences of α and β chains of a wild type TCR capable of specifically binding to SLLMWITQC-HLA A0201 complex, respectively.

### DETAILED DESCRIPTION

Through extensive and intensive researches, the present disclosure obtains a high affinity T-cell receptor (TCR) recognizing SLLMWITQC short peptide (derived from NY-ESO-1 protein) which is presented in the form of peptide-HLA A0201 complex. The high affinity TCR has a mutation in three CDRs of its α chain variable domain:
CDR1α: TSINN
CDR2α: IRSNERE
CDR3α: ATDANGKII; and/or the high affinity TCR has a mutation in three CDRs of its β chain variable domain:
CDR1β: SGHDY
CDR2β: FNNNVP
CDR3β: ASSLGSNEQY; and after the mutation, the affinity and/or binding half-life of the TCR of the present disclosure for SLLMWITQC-HLA A0201 complex is at least twice that of a wild type TCR.

Before the present disclosure is described, it is to be understood that the present disclosure is not limited to the specific methods and experimental conditions described herein, as such methods and conditions may vary. It is also to be understood that the terms used herein are only for the purpose of describing particular embodiments and are not intended to be limitative, and the scope of the present disclosure is only limited by the appended claims.

Unless otherwise defined, all technical and scientific terms used herein have the same meanings as commonly understood by those skilled in the art to which the present disclosure belongs.

Although any methods and materials similar or equivalent to those described in the present disclosure can be used in the practice or testing of the present disclosure, the preferred methods and materials are exemplified herein.

### Term

### T-cell receptor (TCR)

The TCR may be described using the International ImMunoGeneTics Information System (IMGT). A native αβ heterodimeric TCR has an α chain and a β chain. Generally speaking, each chain comprises a variable region, a junction region and a constant region, and typically the β chain also contains a short hypervariable region between the variable region and the junction region. However, the hypervariable region is often considered as part of the junction region. The junction region of the TCR is determined by the unique TRAJ and TRBJ of the IMGT, and the constant region of the TCR is determined by TRAC and TRBC of the IMGT.

Each variable region comprises three complementarity-determining regions (CDRs), CDR1, CDR2 and CDR3, which are chimeric in the framework sequence. In IMGT nomenclature, different numbers of TRAV and TRBV refer to different Vα types and Vβ types, respectively. In the IMGT system, there are the following symbols for an α chain constant domain: TRAC^{∗}01, where "TR" represents a TCR gene, "A" represents an α chain gene, C represents the constant region, and "^{∗}01" represents allele gene 1. There are the following symbols for a β chain constant domain: TRBC1^{∗}01 or TRBC2^{∗}01, where "TR" represents a TCR gene, "B" represents a β chain gene, C represents the constant region, and "^{∗}01" represents allele gene 1. The constant region of the α chain is uniquely defined, and in the form of the β chain, there are two possible constant region genes "C1" and "C2". Those skilled in the art can obtain constant region gene sequences of TCR α and β chains through the disclosed IMGT database.

The α and β chains of the TCR are generally considered as having two "domains" respectively, that is, a variable domain and a constant domain. The variable domain consists of a variable region and a junction region linked to each other. Therefore, in the description and claims of the present application, a "TCR α chain variable domain" refers to TRAV and TRAJ regions linked together. Likewise, a "TCR β chain variable domain" refers to TRBV and TRBD/TRBJ regions linked together. Three CDRs of the TCR α chain variable domain are CDR1α, CDR2α and CDR3α, respectively; and three CDRs of the TCR β chain variable domain are CDR1β, CDR2β and CDR3β, respectively. The framework sequences of TCR variable domains of the present disclosure may be of murine or human origin, preferably of human origin. The constant domain of the TCR comprises an intracellular portion, a transmembrane region and an extracellular portion. To obtain a soluble TCR so as to determine the affinity of the TCR for SLLMWITQC-HLA A0201 complex, the TCR of the present disclosure preferably does not comprise a transmembrane region. More preferably, the amino acid sequence of the TCR of the present disclosure refers to an extracellular amino acid sequence of the TCR.

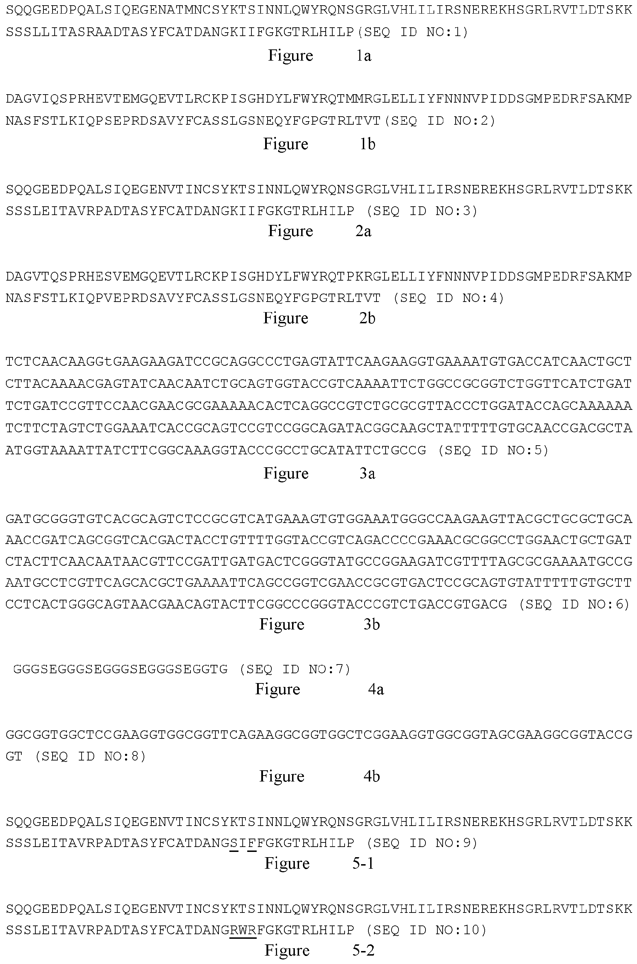

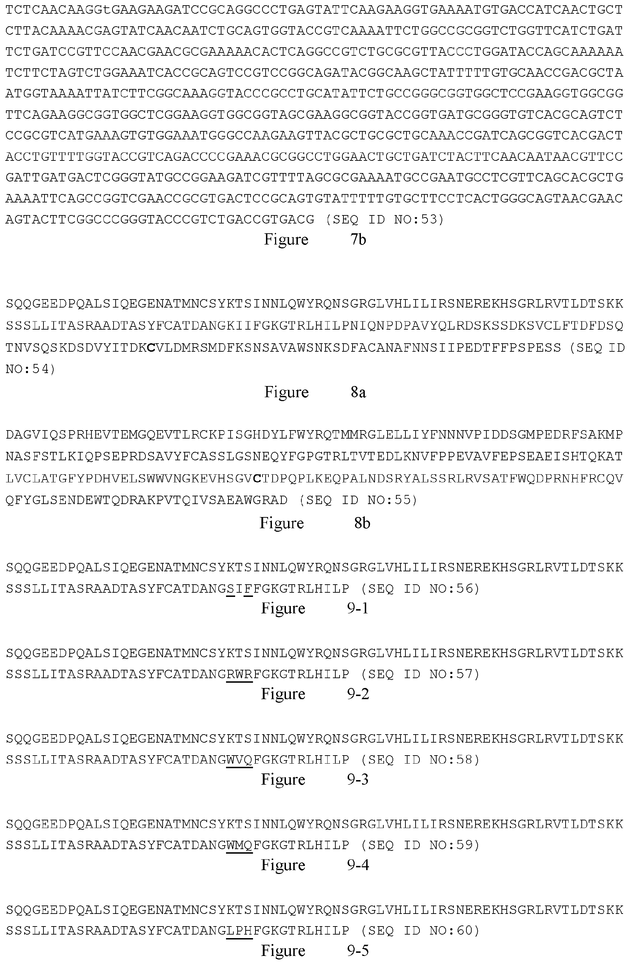

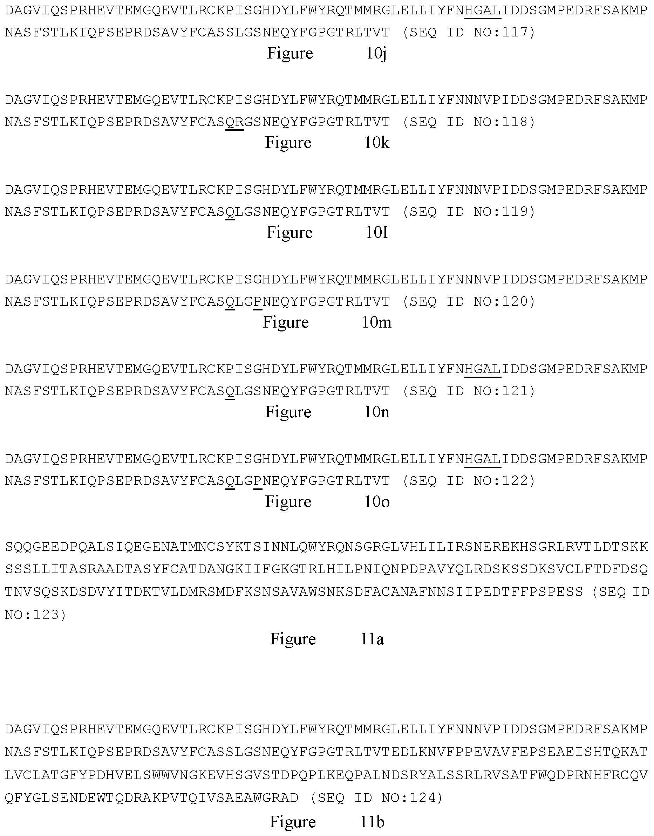

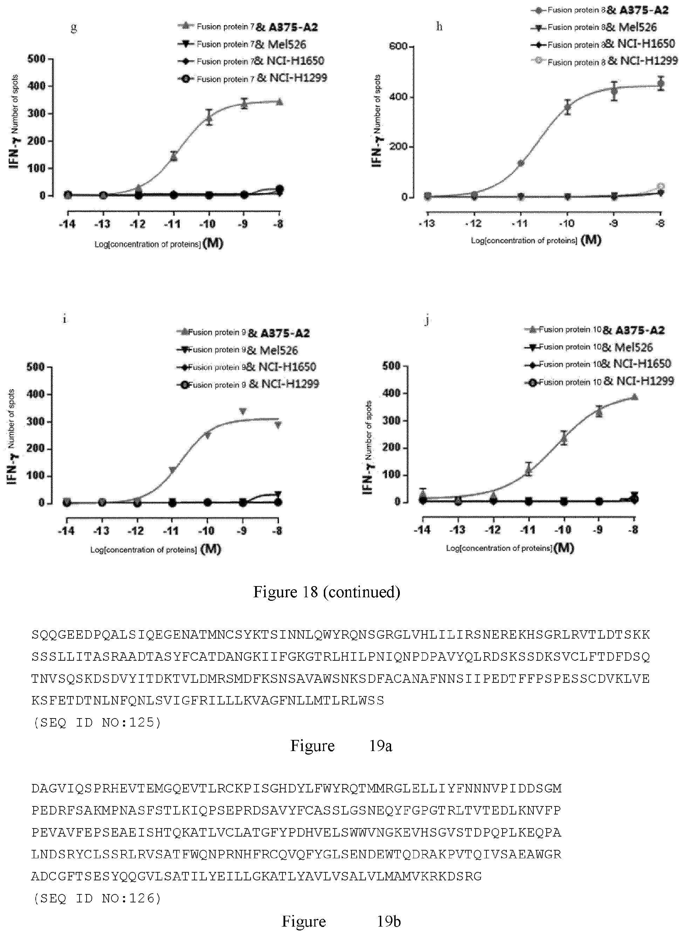

TCR sequences used in the present disclosure are of human origin. The amino acid sequence of the α chain and the extracellular amino acid sequence of the β chain of the "wild type TCR" in the present disclosure are SEQ ID NO: 123 and SEQ ID NO: 124, respectively, as shown in FIGS. 11a and 11b. The amino acid sequences of the α chain and the β chain of a "reference TCR" in the present disclosure are SEQ ID NO: 54 and SEQ ID NO: 55, respectively, as shown in FIGS. 8a and 8b. The amino acid sequences of the α chain and the β chain of the "wild type TCR" in the present disclosure are SEQ ID NO: 125 and SEQ ID NO: 126, respectively, as shown in FIGS. 19a and 19b. In the present disclosure, the amino acid sequences of α and β chain variable domains of the wild type TCR capable of binding to SLLMWITQC-HLA A0201 complex are SEQ ID NO: 1 and SEQ ID NO: 2, respectively, as shown in FIGS. 1a and 1b. In the present disclosure, the terms "polypeptide of the present disclosure", "TCR of the present disclosure" and "T-cell receptor of the present disclosure" are used interchangeably.

### Natural interchain disulfide bond and artificial interchain disulfide bond

A group of disulfide bonds is present between Cα and Cβ chains in the membrane proximal region of a native TCR, which is referred to as "natural interchain disulfide bonds" in the present disclosure. In the present disclosure, an interchain covalent disulfide bond which is artificially introduced and located at a position different from the position of the natural interchain disulfide bond is referred to as "artificial interchain disulfide bond".

For convenience of description, in the present disclosure, the positions of the amino acid sequences of TRAC^{∗}01 and TRBC1^{∗}01 or TRBC2^{∗}01 are sequentially numbered from N-terminal to C-terminal. For example, the 60th amino acid in the order from N-terminal to C-terminal in TRBC1^{∗}01 or TRBC2^{∗}01 is P (proline), which may be described as Pro60 of TRBC1^{∗}01 or TRBC2^{∗}01 exon 1 in the present disclosure and may also be expressed as the amino acid at position 60 of TRBC1^{∗}01 or TRBC2^{∗}01 exon 1. In another example, the 61st amino acid in the order from N-terminal to C-terminal in TRBC1^{∗}01 or TRBC2^{∗}01 is Q (glutamine), which may be described as Gln61 of TRBC1^{∗}01 or TRBC2^{∗}01 exon 1 in the present disclosure and may also be expressed as the amino acid at position 61 of TRBC1^{∗}01 or TRBC2^{∗}01 exon 1, and so on. In the present disclosure, the positions of the amino acid sequences of variable regions TRAV and TRBV are numbered according to the positions listed in the IMGT. For example, the position of an amino acid in TRAV is numbered as 46 in the IMGT, the amino acid is described in the present disclosure as the amino acid at position 46 of TRAV, and so on. In the present disclosure, if the positions of other amino acid sequences are specifically described, the special description shall prevail.

### Tumor

The term "tumor" refers to the inclusion of all types of cancer cell growth or carcinogenic processes, metastatic tissues or malignant transformed cells, tissues or organs, regardless of pathological type or stage of infection. Examples of tumors include, without limitation, solid tumors, soft tissue tumors and metastatic lesions. Examples of solid tumors include malignant tumors of different organ systems, such as sarcoma, lung squamous cell carcinoma and cancer. For example, infected prostate, lung, breast, lymph, gastrointestinal (e.g., colon) and genitourinary tract (e.g., kidney, epithelial cells) and pharynx. Lung squamous cell carcinoma includes malignant tumors, for example, most of colon cancer, rectal cancer, renal cell carcinoma, liver cancer, non-small cell lung cancer, small intestine cancer and esophageal cancer. Metastatic lesions of the above cancer may likewise be treated and prevented using the methods and compositions of the present disclosure.

### Detailed description of the present disclosure

As is known to all, an α chain variable domain and a β chain variable domain of a TCR each contain three CDRs, which are similar to the complementarity-determining regions of an antibody. CDR3 interacts with an antigen short peptide, and CDR1 and CDR2 interact with HLA. Therefore, the CDRs of a TCR molecule determine their interaction with an antigen short peptide-HLA complex. The amino acid sequences of the α chain variable domain and the β chain variable domain of a wild type TCR capable of binding a complex of antigen short peptide SLLMWITQC and HLA A0201 (that is, SLLMWITQC-HLA A0201 complex) are SEQ ID NO: 1 and SEQ ID NO: 2, respectively. The wild type TCR has the following CDRs:
CDRs of the α chain variable domain which include CDR1α (TSINNCDR), 2α (IRSNERECDR) and 3α (ATDANGKII) and CDRs of the β chain variable domain which include CDR1β (SGHDY), CDR2β (FNNNVP) and CDR3β (ASSLGSNEQY).

In the present disclosure, a high affinity TCR is obtained through mutations and screening in the preceding CDR regions, where the affinity of the high affinity TCR for SLLMWITQC-HLA A0201 complex is at least twice that of the wild type TCR for SLLMWITQC-HLA A0201 complex.

The present disclosure provides a T-cell receptor (TCR) which has an activity of binding to SLLMWITQC-HLA A0201 complex.

In the present disclosure, the three CDRs of the α chain variable domain (SEQ ID NO: 1) of the wild type TCR, i.e., CDR1, CDR2 and CDR3, are located at positions 27-31, positions 49-55 and positions 90-98 of SEQ ID NO: 1, respectively. Accordingly, amino acid residues are numbered as shown in SEQ ID NO: 1, 27T is T at position 1 of CDR1α, 28S is S at position 2 of CDR1α, 291 is I at position 3 of CDR1α, 30N is N at position 4 of CDR1α, 51S is S at position 3 of CDR2α, 52N is N at position 4 of CDR2α, 53E is E at position 5 of CDR2α, 54R is R at position 6 of CDR2α, 90A is A at position 1 of CDR3α, 91T is T at position 2 of CDR3α, 93A is A at position 4 of CDR3α, 94N is N at position 5 of CDR3α, 95G is G at position 6 of CDR3α, 96K is K at position 7 of CDR3α, 971 is I at position 8 of CDR3α, and 981 is I at position 9 of CDR3α.

Similarly, in the present disclosure, the three CDRs of the β chain variable domain (SEQ ID NO: 2) of the wild type TCR, i.e., CDR1, CDR2 and CDR3, are located at positions 27-31, positions 49-54 and positions 93-102 of SEQ ID NO: 2, respectively. Accordingly, amino acid residues are numbered as shown in SEQ ID NO: 2, 51N is N at position 3 of CDR2β, 52N is N at position 4 of CDR2β, 53V is V at position 5 of CDR2β, 54P is P at position 6 of CDR2β, 93A is A at position 1 of CDR3β, 95S is S at position 3 of CDR3β, 96L is L at position 4 of CDR3β, 97G is G at position 5 of CDR3β, 98S is S at position 6 of CDR3β, 99N is N at position 7 of CDR3β, 100E is E at position 8 of CDR3β, 101Q is Q at position 9 of CDR3β, and 102Y is Y at position 10 of CDR3β.

The present disclosure provides a TCR having a property of binding to SLLMWITQC-HLA A0201 complex and comprising an α chain variable domain and a β chain variable domain, wherein the TCR has a mutation in the α chain variable domain shown in SEQ ID NO: 1, and the site of the mutated amino acid residue includes one or more of 27T, 28S, 291, 30N, 51S, 52N, 53E, 54R, 90A, 91T, 93A, 94N, 95G, 96K, 971 and 98I, wherein the amino acid residues are numbered as shown in SEQ ID NO: 1; and/or the TCR has a mutation in the β chain variable domain shown in SEQ ID NO: 2, and the site of the mutated amino acid residue includes one or more of 51N, 52N, 53V, 54P, 93A, 95S, 96L, 97G, 98S, 99N, 100E, 101Q and 102Y, wherein the amino acid residues are numbered as shown in SEQ ID NO: 2.

Preferably, the mutated TCR α chain variable domain comprises one or more amino acid residues selected from the group consisting of: 27G or 27Q or 27V, 28W or 28T or 28N, 29A or 29P, 30Q, 51T, 52G, 53Q, 54A, 90G or 90L or 90M, 91F or 91W or 91Y, 93E or 93H or 93M or 93Q or 93R or 93S, 94A or 94D or 94F or 94H or 94S or 94W, 95A, 96R or 96Q or 96S or 96L or 96T or 96W, 97W or 97V or 97M or 97P and 98F or 98E or 98D or 98H or 98L or 98Q or 98R or 98T or 98Y, wherein the amino acid residues are numbered as shown in SEQ ID NO: 1; and/or the mutated TCR β chain variable domain comprises one or more amino acid residues selected from the group consisting of: 51H, 52G, 53A, 54L, 93S, 95Q, 96R or 96K or 96H or 96Q, 97A, 98A or 98G or 98P, 99G, 100P, 101W and 1021, wherein the amino acid residues are numbered as shown in SEQ ID NO: 2.

More specifically, in the α chain variable domain, specific forms of the mutation include one or more of T27G/Q/V, S28W/T/N, I29A/P, N30Q, S51T, N52G, E53Q, R54A, A90G/L/M, T91F/W/Y, A93E/H/M/Q/R/S, N94A/D/F/H/S/W, G95A, K96R/Q/S/L/T/W, I97W/V/M/P and I98F/E/D/H/L/Q/R/T/Y; and in the β chain variable domain, specific forms of the mutation include one or more of N51H, N52G, V53A, P54L, A93S, S95Q, L96R/K/H/Q, G97A, S98A/G/P, N99G, E100P, Q101W and Y102I.

Further, the TCR of the present disclosure is an αβ heterodimeric TCR, and the α chain variable domain of the TCR comprises an amino acid sequence having at least 85%, preferably at least 90%, more preferably at least 92%, most preferably at least 94% (for example, may be at least 88%, 89%, 90%, 91%, 92%, 93%, 94%, 95%, 96%, 97%, 98%, 99%) of sequence homology with the amino acid sequence as shown in SEQ ID NO: 1; and/or the β chain variable domain of the TCR comprises an amino acid sequence having at least 90%, preferably at least 92%, more preferably at least 94%, most preferably at least 97% (for example, may be at least 91%, 92%, 93%, 94%, 95%, 96%, 97%, 98%, 99%) of sequence homology with the amino acid sequence as shown in SEQ ID NO: 2.

Further, the TCR of the present disclosure is a single chain TCR, and the α chain variable domain of the TCR comprises an amino acid sequence having at least 85%, preferably at least 90%, more preferably at least 92%, most preferably at least 94% (for example, may be at least 88%, 89%, 90%, 91%, 92%, 93%, 94%, 95%, 96%, 97%, 98%, 99%) of sequence homology with the amino acid sequence as shown in SEQ ID NO: 3; and/or the β chain variable domain of the TCR comprises an amino acid sequence having at least 90%, preferably at least 92%, more preferably at least 94%, most preferably at least 97% (for example, may be at least 91%, 92%, 93%, 94%, 95%, 96%, 97%, 98%, 99%) of sequence homology with the amino acid sequence as shown in SEQ ID NO: 4.

Preferably, the TCR comprises (i) all or part of a TCR α chain other than its transmembrane domain and (ii) all or part of a TCR β chain other than its transmembrane domain, wherein both of (i) and (ii) comprise a variable domain and at least part of a constant domain of the TCR chain.

According to the site-directed mutagenesis method well known to those skilled in the art, Thr48 of the α chain constant region TRAC^{∗}01 exon 1 of the wild type TCR is mutated to cysteine and Ser57 of the β chain constant region TRBC1^{∗}01 or TRBC2^{∗}01 exon 1 is mutated to cysteine so as to obtain a reference TCR whose amino acid sequences are shown in FIGS. 8a and 8b, respectively, wherein the mutated cysteine residues are indicated by bold letters. The above cysteine substitutions can form an artificial interchain disulfide bond between an α chain constant region and a β chain constant region of the reference TCR to form a more stable soluble TCR, so that it is easier to evaluate the binding affinity and/or binding half-life between the TCR and SLLMWITQC-HLA A0201 complex. It will be appreciated that CDRs of the TCR variable region determine its affinity for pMHC complex so that the above cysteine substitutions in the TCR constant region will not affect the binding affinity and/or binding half-life of the TCR. Therefore, in the present disclosure, the measured binding affinity between the reference TCR and SLLMWITQC-HLA A0201 complex is considered to be the binding affinity between the wild type TCR and SLLMWITQC-HLA A0201 complex. Similarly, if the binding affinity between the TCR of the present disclosure and SLLMWITQC-HLA A0201 complex is determined to be at least 10 times the binding affinity between the reference TCR and SLLMWITQC-HLA A0201 complex, the binding affinity between the TCR of the present disclosure and SLLMWITQC-HLA A0201 complex is at least 10 times the binding affinity between the wild type TCR and SLLMWITQC-HLA A0201 complex.

The binding affinity (inversely proportional to a dissociation equilibrium constant K_{D}) and the binding half-life (expressed as T_{1/2}) can be determined by any suitable method. For example, the detection is performed using surface plasmon resonance technology. It is to be understood that doubling of the binding affinity of the TCR will halve K_{D}. T_{1/2} is calculated as In2 divided by a dissociation rate (K_{off}). Therefore, doubling of T_{1/2} will halve K_{off}. Preferably, the binding affinity or binding half-life of a given TCR is detected several times, for example, three or more times by using the same test protocol, and an average of the results is taken. In a preferred embodiment, the affinity of a soluble TCR is detected under the conditions of a temperature of 25°C and a pH of 7.1-7.5 by a surface plasmon resonance method in the Examples herein. The dissociation equilibrium constant K_{D} of the reference TCR to SLLMWITQC-HLA A0201 complex is detected to be 4.3E-05M, that is, 43 µM by the method. In the present disclosure, the dissociation equilibrium constant K_{D} of the wild type TCR to SLLMWITQC-HLA A0201 complex is also considered to be 43 µM. Since doubling of the affinity of the TCR will halve K_{D}, if the dissociation equilibrium constant K_{D} of a high affinity TCR to SLLMWITQC-HLA A0201 complex is detected to be 4.3E-06M, that is, 4.3 µM, the affinity of the high affinity TCR for SLLMWITQC-HLA A0201 complex is 10 times that of the wild type TCR for SLLMWITQC-HLA A0201 complex. Those skilled in the art are familiar with the conversion relationship between K_{D} value units, that is, 1 M = 1000 µM, 1 µM = 1000 nM, and 1 nM = 1000 pM.

In a preferred embodiment of the present disclosure, the affinity of the TCR for SLLMWITQC-HLA A0201 complex is at least twice, preferably at least five times, more preferably at least ten times that of the wild type TCR.

In another preferred embodiment, the affinity of the TCR for SLLMWITQC-HLA A0201 complex is at least 50 times, preferably at least 100 times, more preferably at least 500 times, most preferably at least 1000 times that of the wild type TCR.

In another preferred embodiment, the affinity of the TCR for SLLMWITQC-HLA A0201 complex is at least 10⁴ times, preferably at least 10⁵ times, more preferably at least 10⁶ times that of the wild type TCR.

Specifically, the dissociation equilibrium constant K_{D} of the TCR to SLLMWITQC-HLA A0201 complex is K_{D} ≤ 20 µM.

In another preferred embodiment, the dissociation equilibrium constant K_{D} of the TCR to SLLMWITQC-HLA A0201 complex is 5 µM ≤ K_{D} ≤ 10 µM, preferably 0.1 µM ≤ K_{D} ≤ 1 µM, more preferably 1 nM ≤ K_{D} ≤ 100 nM.

In another preferred embodiment, the dissociation equilibrium constant K_{D} of the TCR to SLLMWITQC-HLA A0201 complex is 100 pM ≤ K_{D} ≤ 1000 pM, preferably 10 pM ≤ K_{D} ≤ 100 pM.

Mutations can be carried out by any suitable method including, but not limited to, those based on the polymerase chain reaction (PCR), restriction enzyme-based cloning or a linkage-independent cloning (LIC) method. These methods are described in detail in many standard molecular biology textbooks. More details about polymerase chain reaction (PCR) mutagenesis and restriction enzyme-based cloning can be found in Sambrook and Russell, (2001) Molecular Cloning-A Laboratory Manual (Third Edition) CSHL Publishing house. More information about the LIC method can be found in Rashtchian, (1995) Curr Opin Biotechnol 6(1): 30-6.

A method for producing the TCR of the present disclosure may be, but not limited to, screening a TCR having high affinity for SLLMWITQC-HLA-A0201 complex from a diverse library of phage particles displaying such TCRs, as described in a literature (Li, et al. (2005) Nature Biotech 23(3): 349-354).

It will be appreciated that genes expressing amino acids of α and β chain variable domains of the wild type TCR or genes expressing amino acids of α and β chain variable domains of a slightly modified wild type TCR can both be used for preparing template TCRs. Changes necessary to produce the high affinity TCR of the present disclosure are then introduced into the DNA encoding the variable domain of the template TCR.

The high affinity TCR of the present disclosure comprises one of amino acid sequences of the α chain variable domain as shown in SEQ ID NOs: 56-107 and/or one of amino acid sequences of the β chain variable domain as shown in SEQ ID NOs: 108-122. Therefore, a TCR α chain containing the amino acid sequence (SEQ ID NO: 1) of the α chain variable domain of the wild type TCR may bind to a TCR β chain comprising one of SEQ ID NOs: 108-122 to form a heterodimeric TCR or single chain TCR molecule. Alternatively, a TCR β chain containing the amino acid sequence (SEQ ID NO: 2) of the β variable domain of the wild type TCR may bind to a TCR α chain comprising one of SEQ ID NOs: 56-107 to form a heterodimeric TCR or single chain TCR molecule. Alternatively, a TCR α chain comprising one of amino acid sequences of the TCR α chain variable domain as shown in SEQ ID NOs: 56-107 may bind to a TCR β chain comprising one of amino acid sequences of the TCR β chain variable domain as shown in SEQ ID NOs: 108-122 to form a heterodimeric TCR or single chain TCR molecule. In the present disclosure, the amino acid sequences of the α chain variable domain and the β chain variable domain which form the heterodimeric TCR molecule are preferably selected from Table 1.

**Table 1**

| TCR No. | Sequence of a chain variable domain SEQ ID NO: | Sequence of β chain variable domain SEQ ID NO: |
|---|---|---|
| 27 | 82 | 2 |
| 22 | 77 | 2 |
| 1 | 56 | 2 |
| 2 | 57 | 2 |
| 3 | 58 | 2 |
| 4 | 59 | 2 |
| 5 | 60 | 2 |
| 6 | 61 | 2 |
| 7 | 62 | 2 |
| 8 | 63 | 2 |
| 9 | 64 | 2 |
| 10 | 65 | 2 |
| 11 | 66 | 2 |
| 12 | 67 | 2 |
| 13 | 68 | 2 |
| 14 | 69 | 2 |
| 15 | 70 | 2 |
| 16 | 71 | 2 |
| 17 | 72 | 2 |
| 18 | 73 | 2 |
| 19 | 74 | 2 |
| 20 | 75 | 2 |
| 21 | 76 | 2 |
| 23 | 78 | 2 |
| 24 | 79 | 2 |
| 25 | 80 | 2 |
| 26 | 81 | 2 |
| 28 | 83 | 2 |
| 29 | 84 | 2 |
| 30 | 85 | 2 |
| 31 | 86 | 2 |
| 32 | 87 | 2 |
| 33 | 88 | 2 |
| 34 | 89 | 2 |
| 35 | 1 | 108 |
| 36 | 1 | 109 |
| 37 | 1 | 110 |
| 38 | 1 | 111 |
| 39 | 1 | 112 |
| 40 | 1 | 113 |
| 41 | 1 | 114 |
| 42 | 1 | 115 |
| 43 | 1 | 116 |
| 44 | 90 | 2 |
| 45 | 91 | 2 |
| 46 | 1 | 117 |
| 47 | 1 | 118 |
| 48 | 85 | 117 |
| 49 | 84 | 117 |
| 50 | 85 | 119 |
| 51 | 85 | 120 |
| 52 | 92 | 2 |
| 53 | 93 | 2 |
| 54 | 94 | 117 |
| 55 | 95 | 117 |
| 56 | 96 | 117 |
| 57 | 97 | 117 |
| 58 | 94 | 121 |
| 59 | 95 | 121 |
| 60 | 96 | 121 |
| 61 | 97 | 121 |
| 62 | 94 | 122 |
| 63 | 95 | 122 |
| 64 | 96 | 122 |
| 65 | 97 | 122 |
| 66 | 98 | 2 |
| 67 | 98 | 117 |
| 68 | 98 | 119 |
| 69 | 98 | 120 |
| 70 | 99 | 117 |
| 71 | 100 | 117 |
| 72 | 101 | 117 |
| 73 | 102 | 117 |
| 74 | 99 | 121 |
| 75 | 100 | 121 |
| 76 | 101 | 121 |
| 77 | 102 | 121 |
| 78 | 99 | 122 |
| 79 | 100 | 122 |
| 80 | 101 | 122 |
| 81 | 102 | 122 |
| 82 | 103 | 2 |
| 83 | 103 | 117 |
| 84 | 103 | 119 |
| 85 | 103 | 120 |
| 86 | 104 | 117 |
| 87 | 105 | 117 |
| 88 | 106 | 117 |
| 89 | 107 | 117 |
| 90 | 104 | 121 |
| 91 | 105 | 121 |
| 92 | 106 | 121 |
| 93 | 107 | 121 |
| 94 | 104 | 122 |
| 95 | 105 | 122 |
| 96 | 106 | 122 |
| 97 | 107 | 122 |

Based on the object of the present disclosure, the TCR of the present disclosure is a moiety having at least one TCR α and/or TCR β chain variable domain. Such TCRs generally comprise both the TCR α chain variable domain and the TCR β chain variable domain. They may be αβ heterodimers or in a single chain form or any other stable forms. In adoptive immunotherapy, the full length chain of the αβ heterodimeric TCR (including the cytoplasmic and transmembrane domains) can be transfected. The TCR of the present disclosure can be used as a targeting agent for delivering a therapeutic agent to an antigen-presenting cell or be used in combination with other molecules to prepare a bifunctional polypeptide to direct effector cells when the TCR is preferably in a soluble form.

As for stability, it is disclosed in the existing art that a soluble and stable TCR molecule can be obtained by introducing an artificial interchain disulfide bond between the α and β chain constant domains of a TCR, as described in PCT/CN2015/093806. Therefore, the TCR of the present disclosure may be a TCR with an artificial interchain disulfide bond introduced between the residues of its α and β chain constant domains. Cysteine residues form an artificial interchain disulfide bond between the α and β chain constant domains of the TCR. Cysteine residues can replace other amino acid residue at suitable positions of a native TCR to form an artificial interchain disulfide bond. For example, Thr48 of TRAC^{∗}01 exon 1 and Ser57 of TRBC1^{∗}01 or TRBC2^{∗}01 exon 1 can be replaced to form a disulfide bond. Other sites for introducing a cysteine residue to form a disulfide bond may be: Thr45 of TRAC^{∗}01 exon 1 and Ser77 of TRBC1^{∗}01 or TRBC2^{∗}01 exon 1; Tyr10 of TRAC^{∗}01 exon 1 and Ser17 of TRBC1^{∗}01 or TRBC2^{∗}01 exon 1; Thr45 of TRAC^{∗}01 exon 1 and Asp59 of TRBC1^{∗}01 or TRBC2^{∗}01 exon 1; Ser15 of TRAC^{∗}01 exon 1 and Glu15 of TRBC1^{∗}01 or TRBC2^{∗}01 exon 1; Arg53 of TRAC^{∗}01 exon 1 and Ser54 of TRBC1^{∗}01 or TRBC2^{∗}01 exon 1; Pro89 of TRAC^{∗}01 exon 1 and Ala19 of TRBC1^{∗}01 or TRBC2^{∗}01 exon 1; or Tyr10 of TRAC^{∗}01 exon 1 and Glu20 of TRBC1^{∗}01 or TRBC2^{∗}01 exon 1. That is, cysteine residues replace any group of the above-mentioned sites in α and β chain constant domains. A maximum of 15, or a maximum of 10, or a maximum of 8 or fewer amino acids may be truncated at one or more C-termini of the constant domain of the TCR of the present disclosure such that it does not include cysteine residues to achieve the purpose of deleting a natural interchain disulfide bond, or the cysteine residues forming a natural interchain disulfide bond can also be mutated to another amino acid for achieving the above purpose.

As described above, the TCR of the present disclosure may comprise an artificial interchain disulfide bond introduced between residues of its α and β chain constant domains. It is to be noted that the introduced artificial disulfide bond as described above can be contained or not contained between the constant domains, and the TCR of the present disclosure may contain a TRAC constant domain sequence and a TRBC1 or TRBC2 constant domain sequence. The TRAC constant domain sequence and the TRBC1 or TRBC2 constant domain sequence of the TCR can be linked by a natural interchain disulfide bond present in the TCR.

Additionally, as for stability, it is also disclosed in a patent literature PCT/CN2016/077680 that the introduction of an artificial interchain disulfide bond between the α chain variable region and the β chain constant region of a TCR can significantly improve the stability of the TCR. Therefore, an artificial interchain disulfide bond may be contained between an α chain variable region and a β chain constant region of the high affinity TCR of the present disclosure. Specifically, cysteine residues forming an artificial interchain disulfide bond between the α chain variable region and the β chain constant region of the TCR replace: an amino acid at position 46 of TRAV and an amino acid at position 60 of TRBC1^{∗}01 or TRBC2^{∗}01 exon 1; an amino acid at position 47 of TRAV and an amino acid at position 61 of TRBC1^{∗}01 or TRBC2^{∗}01 exon 1; an amino acid at position 46 of TRAV and an amino acid at position 61 of TRBC1^{∗}01 or TRBC2^{∗}01 exon 1; or an amino acid at position 47 of TRAV and an amino acid at position 60 of TRBC1^{∗}01 or TRBC2^{∗}01 exon 1. Preferably, such a TCR may comprise (i) all or part of a TCR α chain other than its transmembrane domain and (ii) all or part of a TCR β chain other than its transmembrane domain, wherein both of (i) and (ii) comprise a variable domain and at least part of a constant domain of the TCR chain, and the α chain and the β chain form a heterodimer. More preferably, such TCR may comprise an α chain variable domain, a β chain variable domain and all or part of a β chain constant domain other than the transmembrane domain, which, however, does not comprise an α chain constant domain, and the α chain variable domain and the β chain of the TCR form a heterodimer.

As for stability, in another aspect, the TCR of the present disclosure also includes a TCR having a mutation in its hydrophobic core region, and these mutations in the hydrophobic core region are preferably mutations capable of increasing the stability of the TCR of the present disclosure, as described in WO 2014/206304. Such a TCR can have mutations at the following positions in the variable domain hydrophobic core: (α and/or β chain) variable region amino acids at positions 11, 13, 19, 21, 53, 76, 89, 91, 94 and/or α chain J gene (TRAJ) short peptide amino acids at reciprocal positions 3, 5, 7 and/or β chain J gene (TRBJ) short peptide amino acids at reciprocal positions 2, 4, 6, wherein the positions in an amino acid sequence are numbered according to the position numbers listed in the International ImMunoGeneTics Information System (IMGT). Those skilled in the art know the preceding IMGT and can obtain the position numbers of amino acid residues of different TCRs in the IMGT based on the database.

More specifically, in the present disclosure, a TCR having a mutation in its hydrophobic core region may be a high-stability single chain TCR consisting of TCR α and β chain variable domains linked by a flexible peptide chain. CDRs in the variable region of the TCR determine the affinity of the TCR for a short peptide-HLA complex, and mutations in a hydrophobic core can increase the stability of the TCR without affecting the affinity of the TCR for the short peptide-HLA complex. It is to be noted that the flexible peptide chain in the present disclosure may be any peptide chain suitable for linking TCR α and β chain variable domains. A template chain constructed in Example 1 of the present disclosure for screening high affinity TCRs is the preceding high-stability single chain TCR containing mutations in the hydrophobic core. The affinity between a TCR and SLLMWITQC-HLA-A0201 complex can be more easily evaluated by using a TCR with higher stability.

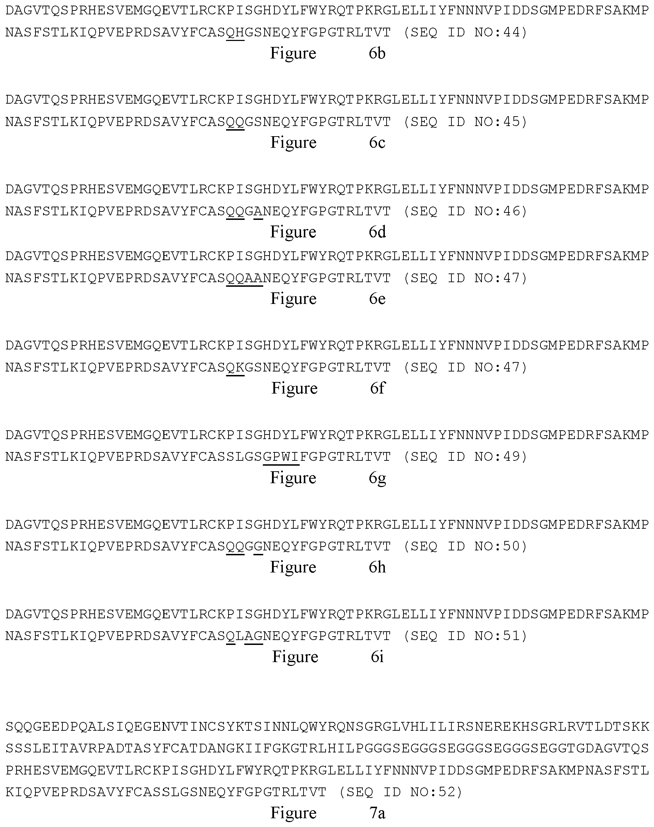

The CDRs of an α chain variable domain and a β chain variable domain of the single chain template TCR are identical to the CDRs of the wild type TCR. That is, three CDRs of the α chain variable domain are CDR1α: TSINN, CDR2α: IRSNERE, and CDR3α: ATDANGKII, respectively, and three CDRs of the β chain variable domain are CDR1β: SGHDY, CDR2β: FNNNVP, and CDR3β: ASSLGSNEQY, respectively. The amino acid sequence (SEQ ID NO: 52) and the nucleotide sequence (SEQ ID NO: 53) of the single chain template TCR are shown in FIGS. 7a and 7b, respectively, thereby screening a single chain TCR consisting of an α chain variable domain and a β chain variable domain and having high affinity for SLLMWITQC-HLA A0201 complex.

In the present disclosure, a CDR of the α chain variable domain (SEQ ID NO: 3) of the single chain template TCR, i.e., CDR3, is located at positions 90-98 of SEQ ID NO: 3. Accordingly, amino acid residues are numbered as shown in SEQ ID NO: 3, 90A is A at position 1 of CDR3α, 91T is T at position 2 of CDR3α, 93A is A at position 4 of CDR3α, 94N is N at position 5 of CDR3α, 95G is G at position 6 of CDR3α, 96K is K at position 7 of CDR3α, 971 is I at position 8 of CDR3α, and 981 is I at position 9 of CDR3α.

Similarly, in the present disclosure, a CDR of the β chain variable domain (SEQ ID NO: 4) of the single chain template TCR, i.e., CDR3, is located at positions 93-102 of SEQ ID NO: 2. Accordingly, amino acid residues are numbered as shown in SEQ ID NO: 2, 93A is A at position 4 of CDR3α, 94N is N at position 5 of CDR3α, 95G is G at position 6 of CDR3α, 96K is K at position 7 of CDR3α, 971 is I at position 8 of CDR3α, and 981 is I at position 9 of CDR3α.

In the present disclosure, some αβ heterodimers having high affinity for SLLMWITQC-HLA-A0201 complex are obtained by transferring the CDRs of α and β chain variable domains of the screened high affinity single chain TCR to the corresponding positions of the α chain variable domain (SEQ ID NO: 1) and the β chain variable domain (SEQ ID NO: 2) of the wild type TCR, and other αβ heterodimers are obtained through artificial combination of the screened mutation sites.

The high affinity TCR of the present disclosure also comprises one of amino acid sequences of the α chain variable domain as shown in SEQ ID NOs: 9-42 or one of amino acid sequences of the β chain variable domain as shown in SEQ ID NOs: 43-51. Therefore, the α chain variable domain (SEQ ID NO: 3) of the preceding high-stability single chain TCR as a template chain may bind to one of the amino acid sequences of the TCR β chain variable domain as shown in SEQ ID NOs: 43-51 to form a single chain TCR molecule. Alternatively, the β chain variable domain (SEQ ID NO: 4) of the preceding high-stability single chain TCR as a template chain may bind to one of the amino acid sequences of the TCR α chain variable domain as shown in SEQ ID NOs: 9-42 to form a single chain TCR molecule. Alternatively, one of SEQ ID NOs: 9-42 of the TCR α chain variable domain may bind to one of SEQ ID NOs: 43-51 of the TCR β chain variable domain to form a single chain TCR molecule. In the present disclosure, the amino acid sequences of the α chain variable domain and the β chain variable domain of the high affinity single chain TCR molecule are preferably selected from Table 2.

**Table 2**

| TCR No. | Sequence of a chain variable domain SEQ ID NO: | Sequence of β chain variable domain SEQ ID NO: |
|---|---|---|
| s-27 | 35 | 4 |
| s-22 | 30 | 4 |
| s-1 | 9 | 4 |
| s-2 | 10 | 4 |
| s-3 | 11 | 4 |
| s-4 | 12 | 4 |
| s-5 | 13 | 4 |
| s-6 | 14 | 4 |
| s-7 | 15 | 4 |
| s-8 | 16 | 4 |
| s-9 | 17 | 4 |
| s-10 | 18 | 4 |
| s-11 | 19 | 4 |
| s-12 | 20 | 4 |
| s-13 | 21 | 4 |
| s-14 | 22 | 4 |
| s-15 | 23 | 4 |
| s-16 | 24 | 4 |
| s-17 | 25 | 4 |
| s-18 | 26 | 4 |
| s-19 | 27 | 4 |
| s-20 | 28 | 4 |
| s-21 | 29 | 4 |
| s-23 | 31 | 4 |
| s-24 | 32 | 4 |
| s-25 | 33 | 4 |
| s-26 | 34 | 4 |
| s-28 | 36 | 4 |
| s-29 | 37 | 4 |
| s-30 | 38 | 4 |
| s-31 | 39 | 4 |
| s-32 | 40 | 4 |
| s-33 | 41 | 4 |
| s-34 | 42 | 4 |
| s-35 | 3 | 43 |
| s-36 | 3 | 44 |
| s-37 | 3 | 45 |
| s-38 | 3 | 46 |
| s-39 | 3 | 47 |
| s-40 | 3 | 48 |
| s-41 | 3 | 49 |
| s-42 | 3 | 50 |
| s-43 | 3 | 51 |

The TCR of the present disclosure can also be provided in the form of a multivalent complex. The multivalent TCR complex of the present disclosure comprises a polymer formed by combining two, three, four or more TCRs of the present disclosure, for example, a tetrameric domain of p53 can be used to produce a tetramer. Alternatively, multiple TCRs of the present disclosure can be combined with another molecule to form a complex. The TCR complex of the present disclosure can be used to track or target cells that present a particular antigen *in vitro* or *in vivo* or produce intermediates of other multivalent TCR complexes with such uses.

The TCR of the present disclosure may be used alone or combined with a conjugate in a covalent manner or another manner, preferably in the covalent manner. The conjugate includes a detectable label (for diagnostic purposes, wherein the TCR is used to detect the presence of a cell presenting SLLMWITQC-HLA-A0201 complex), a therapeutic agent, a protein kinase (PK) modified moiety or a combination of any of the preceding substances.

Detectable labels for diagnostic purposes include, but are not limited to, fluorescent or luminescent labels, radioactive labels, magnetic resonance imaging (MRI) or electron computed tomography (CT) contrast agents or enzymes capable of producing detectable products. Therapeutic agents that can be combined with or coupled to the TCR of the present disclosure include, but are not limited to: 1. radionuclides (Koppe et al., 2005, Cancer metastasis reviews 24, 539); 2. biotoxin (Chaudhary et al., 1989, Nature 339, 394; Epel et al., 2002, Cancer Immunology and Immunotherapy 51, 565); 3. cytokines such as IL-2 (Gillies et al., 1992, Proceedings of the National Academy of Sciences of the United States of America (PNAS) 89, 1428; Card et al., 2004, Cancer Immunology and Immunotherapy 53, 345; Halin et al., 2003, Cancer Research 63, 3202); 4. antibody Fc fragments (Mosquera et al., 2005, the Journal Of Immunology 174, 4381); 5. antibody scFv fragments (Zhu et al., 1995, International Journal of Cancer 62, 319); 6. gold nanoparticles/nanorods (Lapotko et al., 2005, Cancer letters 239, 36; Huang et al., 2006, Journal of the American Chemical Society 128, 2115); 7. viral particles (Peng et al., 2004, Gene therapy 11, 1234); 8. liposomes (Mamot et al., 2005, Cancer research 65, 11631); 9. nanomagnetic particles; 10. prodrug activating enzymes (e.g., DT-diaphorase (DTD) or biphenyl hydrolase-like protein (BPHL); 11. chemotherapeutic agents (e.g., cisplatin) or any form of nanoparticles, and the like.

An antibody binding to the TCR of the present disclosure or a fragment thereof includes an anti-T cell or an NK-cell determining antibody, such as an anti-CD3 or anti-CD28 or anti-CD16 antibody. The above antibody or a fragment thereof binds to the TCR, which can direct effector cells to better target target cells. In a preferred embodiment, the TCR of the present disclosure binds to an anti-CD3 antibody or a functional fragment or variant thereof. Specifically, a fusion molecule of the TCR of the present disclosure and an anti-CD3 single chain antibody comprises a TCR α chain variable domain whose amino acid sequence is selected from the group consisting of SEQ ID NOs: 9-42 and 56-107 and/or a TCR β chain variable domain whose amino acid sequence is selected from the group consisting of SEQ ID NOs: 43-51 and 108-122.

The present disclosure further relates to a nucleic acid molecule encoding the TCR of the present disclosure. The nucleic acid molecule of the present disclosure may be in the form of DNA or RNA. DNA may be a coding strand or a non-coding strand. For example, a nucleic acid sequence encoding the TCR of the present disclosure may be the same as the nucleic acid sequence shown in the Figures of the present disclosure or a degenerate variant thereof. By way of example, the "degenerate variant", as used herein, refers to a nucleic acid sequence which encodes a protein with a sequence of SEQ ID NO: 52 but is different from the sequence of SEQ ID NO: 53.

The full length sequence of the nucleic acid molecule of the present disclosure or a fragment thereof may generally be obtained by, but not limited to, a PCR amplification method, a recombination method or an artificial synthesis method. At present, it has been possible to obtain a DNA sequence encoding the TCR (or a fragment thereof or a derivative thereof) of the present disclosure completely by chemical synthesis. Then, the DNA sequence can be introduced into various existing DNA molecules (or vectors) and cells known in the art.

The present disclosure further relates to a vector comprising the nucleic acid molecule of the present disclosure as well as a host cell genetically engineered using the vector or coding sequence of the present disclosure.

The present disclosure further encompasses an isolated cell, particularly a T cell, which express the TCR of the present disclosure. There are many methods suitable for T cell transfection with DNA or RNA encoding the high affinity TCR of the present disclosure (e.g., Robbins et al., (2008) J. Immunol. 180: 6116-6131). T cells expressing the high affinity TCR of the present disclosure can be used for adoptive immunotherapy. Those skilled in the art can know many suitable methods for adoptive therapy (e.g., Rosenberg et al., (2008) Nat Rev Cancer 8(4): 299-308).

The present disclosure further provides a pharmaceutical composition, which comprise a pharmaceutically acceptable carrier and the TCR of the present disclosure or the TCR complex of the present disclosure or the cell presenting the TCR of the present disclosure.

The present disclosure further provides a method for treating a disease, which comprises administering a subject to be treated with an appropriate amount of the TCR of the present disclosure or the TCR complex of the present disclosure or the cell presenting the TCR of the present disclosure or the pharmaceutical composition of the present disclosure.

It is to be understood that amino acid names herein are identified by internationally accepted single English letters, and the corresponding three-letter abbreviated names of amino acids are: Ala (A), Arg (R), Asn (N), Asp (D), Cys (C), Gln (Q), Glu (E), Gly (G), His (H), Ile (I), Leu (L), Lys (K), Met (M), Phe (F), Pro (P), Ser (S), Thr (T), Trp (W), Tyr (Y), Val (V).

In the present disclosure, Pro60 or 60P represents proline at position 60. Further, regarding the expression of the specific form of a mutation in the present disclosure, for example, "T27G/Q/V" represents that T at position 27 is substituted with G, Q or V. Similarly, "S28W/T/N" represents that S at position 28 is substituted with W, T or N. The rest can be done in the same manner.

In the art, when an amino acid with similar properties is used for substitution, the function of a protein is generally not changed. The addition of one amino acid or multiple amino acids at C-terminal and/or N-terminal generally will not change the structure and function of the protein. Therefore, the TCR of the present disclosure further includes a TCR wherein up to 5, preferably up to 3, more preferably up to 2, most preferably 1 amino acid (especially an amino acid located outside CDRs) of the TCR of the present disclosure is replaced with an amino acid with similar properties and which can still maintain its function.

The present disclosure further includes a TCR slightly modified from the TCR of the present disclosure. The form of a modification (generally without altering a primary structure) includes chemically derived forms of the TCR of the present disclosure, such as acetylation or carboxylation. Modifications also include glycosylation, such as those TCRs produced through glycosylation in the synthesis and processing steps or in the further processing step of the TCR of the present disclosure. Such modification can be accomplished by exposing the TCR to an enzyme for glycosylation (such as a mammalian glycosylation enzyme or a deglycosylation enzyme). The form of the modification further includes sequences having phosphorylated amino acid residues (such as phosphotyrosine, phosphoserine and phosphothreonine). Further included are TCRs that have been modified to enhance their antiproteolytic properties or optimize solubility properties.

The TCR or TCR complex of the present disclosure or the T cell transfected with the TCR of the present disclosure may be provided in a pharmaceutical composition together with a pharmaceutically acceptable carrier. The TCR, multivalent TCR complex or cell of the present disclosure is typically provided as part of a sterile pharmaceutical composition which typically comprises a pharmaceutically acceptable carrier. The pharmaceutical composition may be in any suitable form (depending on the desired method for administration to a patient). The pharmaceutical composition may be provided in a unit dosage form, generally provided in a sealed container and may be provided as part of a kit. Such kits include instructions (which are not necessary). The pharmaceutical composition may include multiple unit dosage forms.

Furthermore, the TCR of the present disclosure may be used alone or in combination with other therapeutic agents (e.g., formulated in the same pharmaceutical composition).

The pharmaceutical composition may also contain a pharmaceutically acceptable carrier. The term "pharmaceutically acceptable carrier" refers to a carrier for administration of a therapeutic agent. The term refers to such pharmaceutical carriers which do not induce the production of antibodies harmful to an individual receiving the composition and which are not excessively toxic after administration. These carriers are well known to those skilled in the art. A full discussion of pharmaceutically acceptable excipients can be found in Remington's Pharmaceutical Sciences (Mack Pub. Co., N. J. 1991). Such carriers include, but are not limited to, saline, buffer, dextrose, water, glycerol, ethanol, adjuvants and combinations thereof.

The pharmaceutically acceptable carrier in the therapeutic composition may contain a liquid such as water, saline, glycerol and ethanol. In addition, auxiliary substances such as wetting or emulsifying agents and pH buffering substances may also be present in these carriers.

Generally, the therapeutic compositions may be formulated as injectables such as liquid solutions or suspensions and may also be formulated as solid forms such as liquid carriers, which may be suitable for being formulated in solutions or suspensions prior to injection.

Once the composition of the present disclosure is formulated, it may be administered by conventional routes including, but not limited to, intraocular, intramuscular, intravenous, subcutaneous, intradermal or topical administration, preferably parenteral administration including subcutaneous, intramuscular or intravenous administration. A subject to be prevented or treated may be an animal, especially a human.

When the pharmaceutical composition of the present disclosure is used for actual treatment, pharmaceutical compositions of various dosage forms may be employed depending on uses, preferably, an injection, an oral preparation or the like.

These pharmaceutical compositions may be formulated by being mixed, diluted or dissolved by conventional methods and, occasionally, be added with suitable pharmaceutical additives such as excipients, disintegrating agents, binders, lubricants, diluents, buffers, isotonicities, preservatives, wetting agents, emulsifiers, dispersing agents, stabilizers and co-solvents, and the formulation process may be carried out in a customary manner depending on the dosage form.

The pharmaceutical composition of the present disclosure may also be administered in the form of a sustained release preparation. For example, the TCR of the present disclosure may be incorporated into a pill or microcapsule with a sustained release polymer as a carrier and then the pill or microcapsule is surgically implanted into the tissue to be treated. Examples of the sustained release polymer include ethylene-vinyl acetate copolymer, polyhydrometaacrylate, polyacrylamide, polyvinylpyrrolidone, methylcellulose, lactic acid polymer, lactic acid-glycolic acid copolymer or the like, preferably a biodegradable polymer such as lactic acid polymer and lactic acid-glycolic acid copolymer.

When the pharmaceutical composition of the present disclosure is used for actual treatment, the amount of the TCR or TCR complex of the present disclosure or the cell presenting the TCR of the present disclosure as an active ingredient may be reasonably determined by a doctor based on the body weight, age, sex and degree of symptoms of each patient to be treated.

### Main advantages of the present disclosure:

(1) The affinity and/or binding half-life of the TCR of the present disclosure for SLLMWITQC-HLA-A0201 complex is at least twice, preferably at least 10 times that of the wild type TCR.
(2) The affinity and/or binding half-life of the TCR of the present disclosure for SLLMWITQC-HLA-A0201 complex is at least 100 times, preferably at least 1000 times, more preferably up to 10⁴ to 10⁶ times that of the wild type TCR.
(3) Effector cells transduced with the high affinity TCR of the present disclosure exhibit a strong killing effect on target cells.

The present disclosure is further illustrated by the following specific examples. It is to be understood that these examples are used only for the purpose of illustration and not intended to limit the scope of the present disclosure. Experimental methods in the following examples which do not specify specific conditions are generally performed under conventional conditions, for example, conditions described in Sambrook and Russell et al., Molecular Cloning-A Laboratory Manual (Third Edition) (2001) CSHL Publishing house or under conditions recommended by the manufacturer. Percentages and parts are by weight unless otherwise stated.

### Materials and Methods

The experimental materials used in the examples of the present disclosure may be commercially available, unless otherwise specified, where *E. coli* DH5α was purchased from Tiangen, *E. coli* BL21 (DE3) was purchased from Tiangen, *E. coli* Tuner (DE3) was purchased from Novagen, and plasmid pET28a was purchased from Novagen.

### Example 1 Generation of stable single chain TCR template chains with mutations in the hydrophobic core

In the present disclosure, a stable single chain TCR molecule consisting of TCR α and β chain variable domains linked by a flexible short peptide (linker) was constructed by a site-directed mutagenesis method according to a patent literature WO2014/206304, where the amino acid sequence and DNA sequence thereof are SEQ ID NO: 52 and SEQ ID NO: 53, respectively, as shown in FIGS. 7a and 7b. The single chain TCR molecule was used as a template for screening a high affinity TCR molecule. The amino acid sequences of an α variable domain (SEQ ID NO: 3) and a β variable domain (SEQ ID NO: 4) of the template chain are shown in FIGS. 2a and 2b; the corresponding DNA sequences are SEQ ID NO: 5 and SEQ ID NO: 6, respectively, as shown in FIGS. 3a and 3b; and the amino acid sequence and DNA sequence of the flexible short peptide (linker) are SEQ ID NO: 7 and SEQ ID NO: 8, respectively, as shown in FIGS. 4a and 4b.

The target gene carrying the template chain was digested with NcoI and NotI and ligated with pET28a vector digested with NcoI and NotI. The ligation product was transferred into *E. coli* DH5α, plated on a kanamycin-containing LB plate, inverted and cultured at 37 °C overnight, and the positive clones were picked for PCR screening. Positive recombinants were sequenced to determine the correct sequence and the recombinant plasmid was extracted and transferred into *E. coli* BL21 (DE3) for expression.

### Example 2 Expression, refolding and purification of the stable single chain TCR constructed in Example 1

All of BL21(DE 3) colonies containing the recombinant plasmid pET28a-template chain prepared in Example 1 were inoculated into the LB medium containing kanamycin and cultured at 37 °C until OD₆₀₀ was 0.6-0.8. IPTG was added to a final concentration of 0.5 mM and cultured at 37 °C for another 4 h. The cell pellets were harvested by centrifugation at 5000 rpm for 15 min, and the cell pellets were lysed with Bugbuster Master Mix (Merck). The inclusion bodies were recovered by centrifugation at 6000 rpm for 15 min, followed by washing with Bugbuster (Merck) to remove cell debris and membrane components. The inclusion bodies were collected by centrifugation at 6000 rpm for 15 min. The inclusion bodies were dissolved in a buffer (20 mM Tris-HCl pH 8.0, 8 M urea), and the insoluble substances were removed by high-speed centrifugation. The supernatant was quantitatively determined by the BCA method and then dispensed and stored at -80 °C until use.

To 5 mg of dissolved single-chain TCR inclusion body protein, 2.5 mL of buffer (6 M Gua-HCl, 50 mM Tris-HCl pH 8.1, 100 mM NaCl, 10 mM EDTA) was added, and then DTT was added to a final concentration of 10 mM and incubated at 37 °C for 30 min. The single chain TCR as treated above was added dropwise to 125 mL of refolding buffer (100 mM Tris-HCl pH 8.1, 0.4 M L-arginine, 5 M urea, 2 mM EDTA, 6.5 mM β-mercapthoethylamine, 1.87 mM cystamine) with a syringe and stirred at 4 °C for 10 min. Then the refolded solution was loaded into a cellulose membrane dialysis bag with a cut-off of 4 kDa, and the dialysis bag was placed in 1 L of pre-cooled water and stirred slowly at 4 °C overnight. After 17 h, the dialysis liquid was changed to 1 L of pre-chilled buffer (20 mM Tris-HCl pH 8.0) and dialysis was continued for 8 h at 4 °C. The dialysis liquid was then replaced with the same fresh buffer and dialysis was continued overnight. After 17 h, the sample was filtered through a 0.45 µm filter, vacuum degassed and purified through an anion exchange column (HiTrap Q HP, GE Healthcare) with a linear gradient elution of 0-1 M NaCl prepared with 20 mM Tris-HCl pH 8.0. The collected fractions were subjected to SDS-PAGE analysis, the fractions containing the single chain TCR were concentrated and further purified by a gel filtration column (Superdex 75 10/300, GE Healthcare), and the target fractions were also subjected to the SDS-PAGE analysis.

The eluted fractions for BIAcore analysis were further tested using gel filtration for purity. The conditions were a chromatographic column Agilent Bio SEC-3 (300 A, ϕ7.8×300 mm), a mobile phase 150 mM phosphate buffer, a flow rate of 0.5 mL/min, a column temperature of 25 °C, and a UV detection wavelength of 214 nm.

### Example 3 Binding characterization

### BIAcore analysis

The binding activity of a TCR molecule to SLLMWITQC-HLA-A0201 complex was detected using the BIAcore T200 real-time analysis system. An anti-streptavidin antibody (GenScript) was added to a coupling buffer (10 mM sodium acetate buffer, pH 4.77), and then the antibody passed through a CM5 chip pre-activated with EDC and NHS to immobilize the antibody on the surface of the chip. The unreacted activated surface was finally blocked with a solution of ethanolamine in hydrochloric acid to complete the coupling process at a coupling level of about 15000 RU. The conditions were a temperature of 25 °C and a pH of 7.1-7.5.

Streptavidin with a low concentration flowed over the surface of the antibody-coated chip, and then SLLMWITQC-HLA-A0201 complex flowed through the detection channel with another channel as a reference channel. 0.05 mM biotin flowed over the chip for 2 min at a flow rate of 10 µL/min, thereby blocking the remaining binding sites for streptavidin. The affinity was determined by a single-cycle kinetic analysis. The TCR was diluted to several different concentrations with HEPES-EP buffer (10 mM HEPES, 150 mM NaCl, 3 mM EDTA, 0.005% P20, pH 7.4) and flowed over the surface of the chip in sequence at a flow rate of 30 µL/min, with a binding time of 120s per injection. After the last injection, the chip was placed for dissociation for 600s. At the end of each round of assay, the chip was regenerated with 10 mM Gly-HCl, pH 1.75. Kinetic parameters were calculated using BIAcore Evaluation software.

The preparation process of the preceding SLLMWITQC-HLA-A0201 complex is described below.

### a. Purification

100 mL of *E. coli* liquid induced to express heavy or light chains was collected and centrifuged at 8000 g for 10 min at 4 °C, and the cells were washed once with 10 mL of PBS and then vigorously shaken in 5 mL of BugBuster Master Mix Extraction Reagents (Merck) for resuspending the cells. The suspension was incubated for 20 min at room temperature and then centrifuged at 6000 g for 15 min at 4 °C. The supernatant was discarded to collect inclusion bodies.

The above inclusion bodies were resuspended in 5 mL of BugBuster Master Mix and incubated vortically at room temperature for 5 min. 30 mL of BugBuster diluted 10 times was added, mixed and centrifuged at 6000 g for 15 min at 4 °C. The supernatant was discarded, and 30 mL of BugBuster diluted 10 times was added to resuspend the inclusion bodies, mixed and centrifuged twice at 6000 g at 4 °C for 15 min. 30 mL of 20 mM Tris-HCl pH 8.0 was added to resuspend the inclusion bodies, mixed and centrifuged at 6000 g at 4 °C for 15 min. Finally, the inclusion bodies were dissolved in 20 mM Tris-HCl 8M urea. The purity of the inclusion bodies was determined by SDS-PAGE and the concentration was measured by the BCA kit.

### b. Refolding

The synthesized short peptide SLLMWITQC (Beijing Saibaisheng Gene Technology Co., Ltd.) was dissolved in DMSO to a concentration of 20 mg/mL. Inclusion bodies of light and heavy chains were solubilized in 8 M urea, 20 mM Tris pH 8.0, 10 mM DTT and further denatured by adding 3 M uanidine hydrochloride, 10 mM sodium acetate, 10 mM EDTA before refolding. SLLMWITQC peptide was added to a refolding buffer (0.4 M L-arginine, 100 mM Tris pH 8.3, 2 mM EDTA, 0.5 mM oxidized glutathione, 5 mM reduced glutathione, 0.2 mM PMSF, cooled to 4 °C ) at 25 mg/L (final concentration). Then, 20 mg/L of light chain and 90 mg/L of heavy chain (final concentration, heavy chains were added in three portions, 8 h/portion) were successively added and refolded at 4 °C for at least three days to completion of refolding, and SDS-PAGE was used to confirm refolding.

### c. Purification upon refolding

The refolding buffer was replaced with 10 volumes of 20 mM Tris pH 8.0 for dialysis, and the buffer was replaced at least twice to fully reduce the ionic strength of the solution. After dialysis, the protein solution was filtered through a 0.45 µm cellulose acetate filter and loaded onto a HiTrap Q HP (GE, General Electric Company) anion exchange column (5 mL bed volume). The protein was eluted with a linear gradient of 0-400 mM NaCl prepared in 20 mM Tris pH 8.0 using Akta Purifier (GE), and pMHC was eluted at approximately 250 mM NaCl. Peak fractions were collected and the purity thereof was detected by SDS-PAGE.

### d. Biotinylation

Purified pMHC molecules were concentrated in a Millipore ultrafiltration tube while the buffer was replaced with 20 mM Tris pH 8.0. Then, biotinylation reagents 0.05 M Bicine pH 8.3, 10 mM ATP, 10 mM MgOAc, 50 µM D -Biotin, 100 µg/ml BirA enzyme (GST-BirA) were added. The resulting mixture was incubated at room temperature overnight, and SDS-PAGE was used to detect the completion of biotinylation.

### e. Purification of the biotinylated complex

The biotinylated and labeled pMHC molecules were concentrated to 1 mL in the Millipore ultrafiltration tube. The biotinylated pMHC was purified by gel permeation chromatography. 1 mL of concentrated biotinylated pMHC molecules was loaded on a HiPrep^{™} 16/60 S200 HR column (GE) pre-equilibrated with filtered PBS using Akta Purifier (GE) and eluted with PBS at a flow rate of 1 mL/min. The biotinylated pMHC molecules were eluted as a single peak at about 55 mL. The protein-containing fractions were combined and concentrated in the Millipore ultrafiltration tube. The concentration of proteins was determined by the BCA method (Thermo), a protease inhibitor cocktail (Roche) was added and the biotinylated pMHC molecules were dispensed and stored at -80 °C.

### Example 4 Generation of a high affinity single chain TCR

Phage display technology is a means to generate high affinity TCR variant libraries for screening high affinity variants. The TCR phage display and screening method described by Li et al. ((2005) Nature Biotech 23(3): 349-354) was applied to the single chain TCR template in Example 1. A library of high affinity TCRs was established by mutating CDRs of the template chain and panned. After several rounds of panning, the phage library can specifically bind to the corresponding antigen, a monoclone was picked, and a sequence analysis was performed.

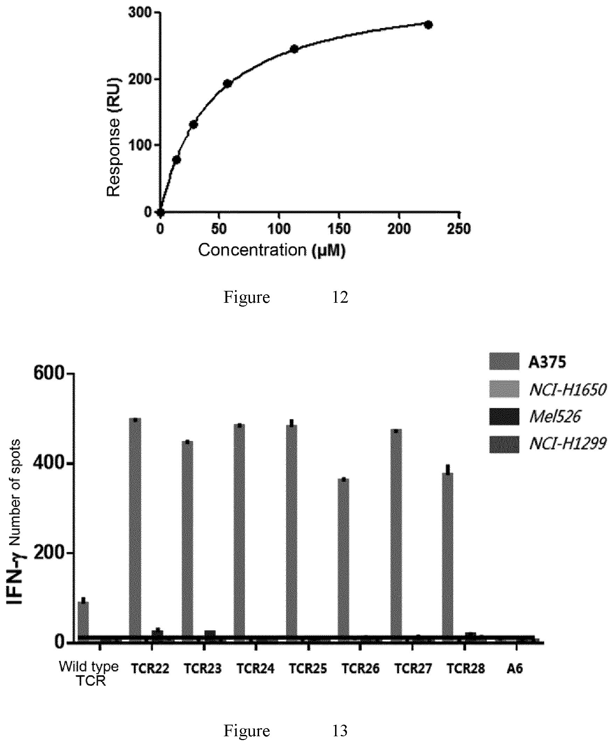

The interaction between a TCR molecule and SLLMWITQC-HLA-A0201 complex was analyzed by the BIAcore method in Example 3, and a high affinity TCR whose affinity and/or binding half-life is at least twice that of a wild type TCR was screened out, that is, the dissociation equilibrium constant K_{D} of the screened high affinity TCR binding to SLLMWITQC-HLA-A0201 complex is less than or equal to a half of the dissociation equilibrium constant K_{D} of the wild type TCR binding to SLLMWITQC-HLA-A0201 complex. The results are shown in Table 3. The K_{D} value of the interaction between the reference TCR and SLLMWITQC-HLA-A0201 complex was detected to be 43 µM by the above method, and the interaction curve is shown in FIG. 12. That is, the K_{D} value of the interaction between the wild type TCR and SLLMWITQC-HLA-A0201 complex is also 43 µM (4.3E-05M).

Specifically, when using the numbers shown in SEQ ID NO: 1, an amino acid at one or more of the following sites in the α chain variable domain of these high affinity TCR mutants is mutated: 27T, 28S, 291, 30N, 51S, 52N, 53E, 54R, 90A, 91T, 93A, 94N, 95G, 96K, 971 and 981, and/or when using the numbers shown in SEQ ID NO: 2, an amino acid at one or more of the following sites in the β chain variable domain of these high affinity TCR mutants is mutated: 51N, 52N, 53V, 54P, 93A, 95S, 96L, 97G, 98S, 99N, 100E, 101Q and 102Y.

More specifically, when using the numbers shown in SEQ ID NO: 1, the α chain variable domain of these high affinity TCRs comprises one or more amino acid residues selected from the group consisting of: 27G or 27Q or 27V, 28W or 28T or 28N, 29A or 29P, 30Q, 51T, 52G, 53Q, 54A, 90G or 90L or 90M, 91F or 91W or 91Y, 93E or 93H or 93M or 93Q or 93R or 93S, 94A or 94D or 94F or 94H or 94S or 94W, 95A, 96R or 96Q or 96S or 96L or 96T or 96W, 97W or 97V or 97M or 97P and 98F or 98E or 98D or 98H or 98L or 98Q or 98R or 98T or 98Y; and/or when using the numbers shown in SEQ ID NO: 2, the β chain variable domain of these high affinity TCRs comprises one or more amino acid residues selected from the group consisting of: 51H, 52G, 53A, 54L, 93S, 95Q, 96R or 96K or 96H or 96Q, 97A, 98A or 98G or 98P, 99G, 100P, 101W and 1021.

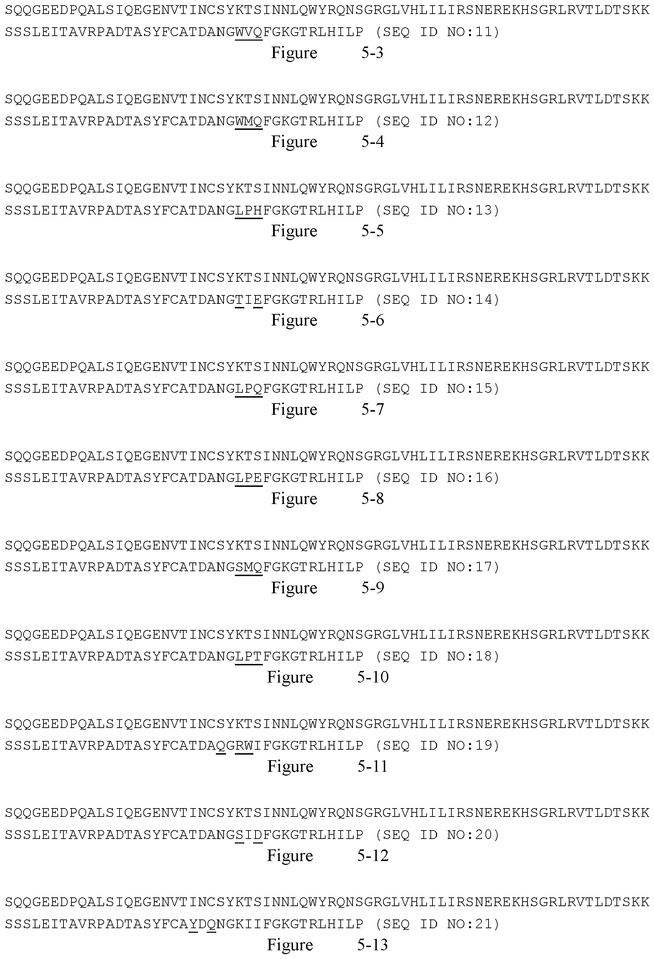

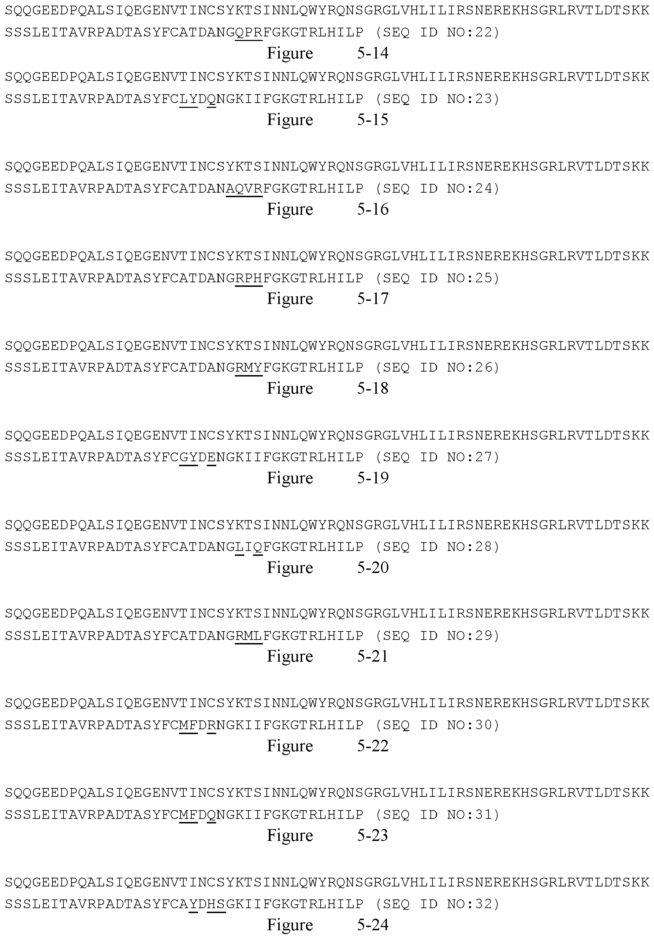

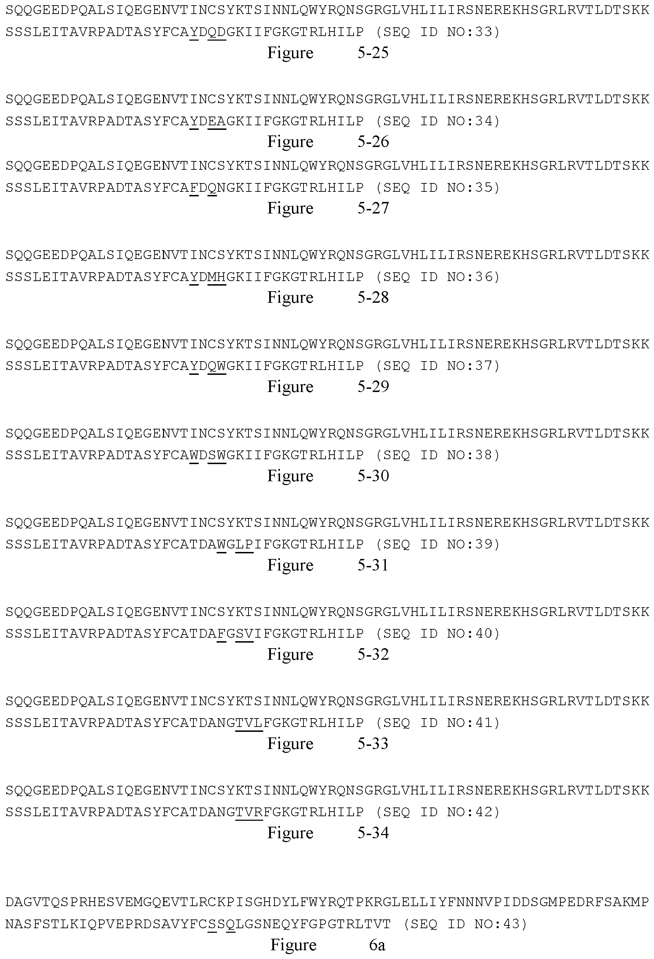

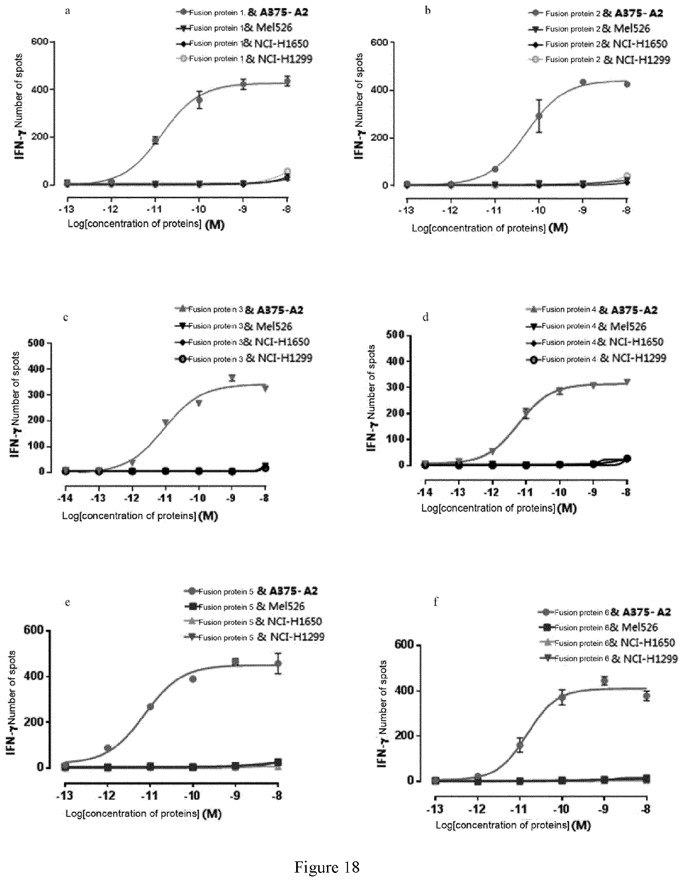

The specific amino acid sequences of α chain variable domains (SEQ ID NOs: 9-42) and β chain variable domains (SEQ ID NOs: 42-51) of the high affinity single chain TCRs are shown in FIGS. 5-1 to 5-34 and FIGS. 6a to 6i, respectively.

**Table 3**

| Single Chain TCR No. | Single Chain TCR Variable Domain (SEQ ID NO.) | | K_{D} (M) |
|---|---|---|---|
| | a | β | |
| s-27 | 35 | 4 | 2.70E-07 |
| s-22 | 30 | 4 | 6.21E-08 |
| s-1 | 9 | 4 | 2.91E-06 |
| s-2 | 10 | 4 | 1.45E-06 |
| s-3 | 11 | 4 | 1.15E-06 |
| s-4 | 12 | 4 | 1.09E-06 |
| s-5 | 13 | 4 | 4.77E-06 |
| s-6 | 14 | 4 | 3.97E-07 |
| s-7 | 15 | 4 | 3.13E-06 |
| s-8 | 16 | 4 | 8.11E-06 |
| s-9 | 17 | 4 | 1.65E-06 |
| s-10 | 18 | 4 | 4.81E-06 |
| s-11 | 19 | 4 | 2.51E-06 |
| s-12 | 20 | 4 | 1.23E-05 |
| s-13 | 21 | 4 | 3.51E-06 |
| s-14 | 22 | 4 | 3.15E-08 |
| s-15 | 23 | 4 | 7.93E-09 |
| s-16 | 24 | 4 | 5.85E-06 |
| s-17 | 25 | 4 | 3.62E-06 |
| s-18 | 26 | 4 | 7.41E-10 |
| s-19 | 27 | 4 | 4.58E-06 |
| s-20 | 28 | 4 | 1.20E-05 |
| s-21 | 29 | 4 | 5.57E-08 |
| s-23 | 31 | 4 | 1.54E-08 |
| s-24 | 32 | 4 | 2.02E-07 |
| s-25 | 33 | 4 | 7.80E-09 |
| s-26 | 34 | 4 | 2.30E-06 |
| s-28 | 36 | 4 | 1.01E-06 |
| s-29 | 37 | 4 | 2.25E-08 |
| s-30 | 38 | 4 | 2.95E-07 |
| s-31 | 39 | 4 | 1.72E-08 |
| s-32 | 40 | 4 | 1.58E-07 |
| s-33 | 41 | 4 | 4.96E-07 |
| s-34 | 42 | 4 | 2.25E-07 |
| s-35 | 3 | 43 | 3.22E-07 |
| s-36 | 3 | 44 | 1.17E-07 |
| s-37 | 3 | 45 | 1.95E-07 |
| s-38 | 3 | 46 | 5.84E-09 |
| s-39 | 3 | 47 | 6.62E-08 |
| s-40 | 3 | 48 | 2.82E-07 |
| s-41 | 3 | 49 | 5.19E-08 |
| s-42 | 3 | 50 | 4.62E-08 |
| s-43 | 3 | 51 | 5.97E-08 |

### Example 5 Generation of a high affinity αβ heterodimeric TCR

The mutations in CDRs of the high affinity single chain TCRs screened in Example 4 were introduced into the corresponding sites of the variable domains of the αβ heterodimeric TCR, and the affinity of the αβ heterodimeric TCR for SLLMWITQC-HLA-A0201 complex was detected by BIAcore. The mutated sites of high affinity were introduced into the above CDRs by a site-directed mutagenesis method well known to those skilled in the art. The amino acid sequences of α and β chain variable domains of the above wild type TCR are shown in FIGS. 1a (SEQ ID NO: 1) and 1b (SEQ ID NO: 2), respectively.

It is to be noted that to obtain a more stable soluble TCR for easier evaluation of the binding affinity and/or binding half-life between the TCR and SLLMWITQC-HLA A0201 complex, the αβ heterodimeric TCR may be a TCR in which a cysteine residue is respectively introduced into α and β chain constant domains to form an artificial interchain disulfide bond. In this example, the amino acid sequences of TCR α and β chains after the introduction of the cysteine residue are shown in FIGS. 8a (SEQ ID NO: 54) and 8b (SEQ ID NO: 55), where the introduced cysteine residues are indicated by bold letters.

According to standard methods described in Molecular Cloning-A Laboratory Manual (Third Edition, Sambrook and Russell), genes of extracellular sequences of TCR α and β chains to be expressed are synthesized and inserted into an expression vector pET28a+ (Novagene) in which the upstream and downstream cloning sites are NcoI and NotI, respectively. Mutations in the CDRs are introduced by overlap PCR well known to those skilled in the art. The inserted fragment was sequenced to confirm that it was correct.

### Example 6 Expression, refolding and purification of the αβ heterodimeric TCR

Expression vectors for TCR α and β chains were transferred into the expression bacteria BL21 (DE3) by chemical transformation, respectively. The bacteria were grown in an LB medium and induced with a final concentration of 0.5 mM IPTG at OD₆₀₀ = 0.6. The inclusion bodies formed after the TCR α and β chains were expressed were extracted by BugBuster Mix (Novagene) and repeatedly washed with a BugBuster solution. The inclusion bodies were finally dissolved in 6 M guanidine hydrochloride, 10 mM dithiothreitol (DTT), 10 mM ethylenediaminetetraacetic acid (EDTA), 20 mM Tris (pH 8.1).

The dissolved TCR α and β chains were rapidly mixed in 5 M urea, 0.4 M arginine, 20 mM Tris (pH 8.1), 3.7 mM cystamine, 6.6 mM β-mercapoethylamine (4 °C) at a mass ratio of 1:1. The final concentration was 60 mg/mL. After mixing, the solution was dialyzed against 10 volumes of deionized water (4 °C). After 12 h, deionized water was replaced with a buffer (20 mM Tris, pH 8.0) and dialysis was continued at 4 °C for 12 h. After completion of the dialysis, the solution was filtered through a 0.45 µM filter and purified through an anion exchange column (HiTrap Q HP, 5 mL, GE Healthcare). The elution peak of the TCR containing the successfully refolded αβ dimer was confirmed by SDS-PAGE gel. The TCR was then further purified by gel permeation chromatography (HiPrep 16/60, Sephacryl S-100 HR, GE Healthcare). The purity of the purified TCR was determined by SDS-PAGE to be greater than 90%, and the concentration thereof was determined by the BCA method.

### Example 7 BIAcore analysis results

The affinity of the αβ heterodimeric TCR with high affinity CDRs introduced for SLLMWITQC-HLA-A0201 complex was detected by the method described in Example 3.

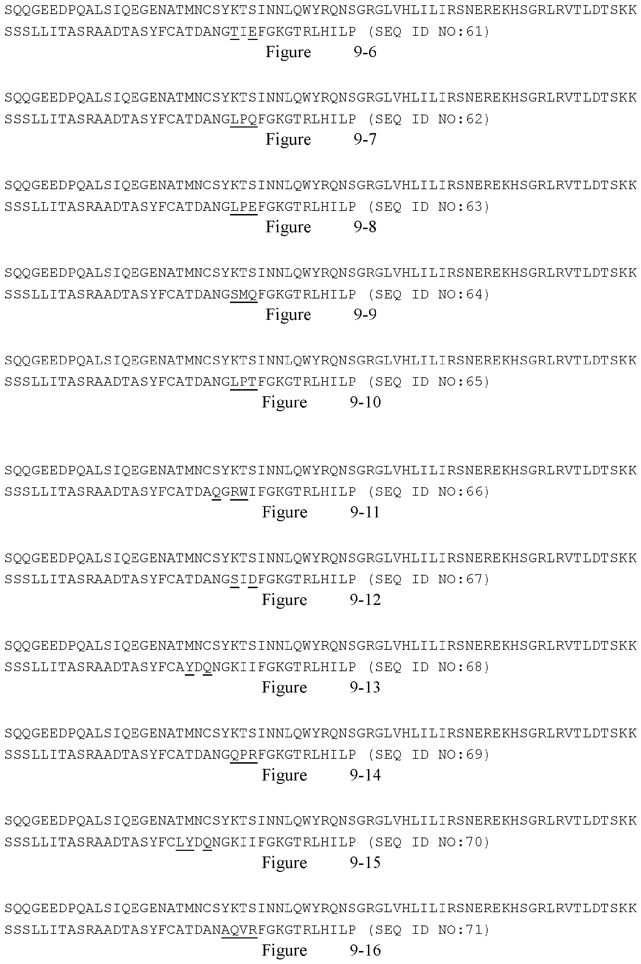

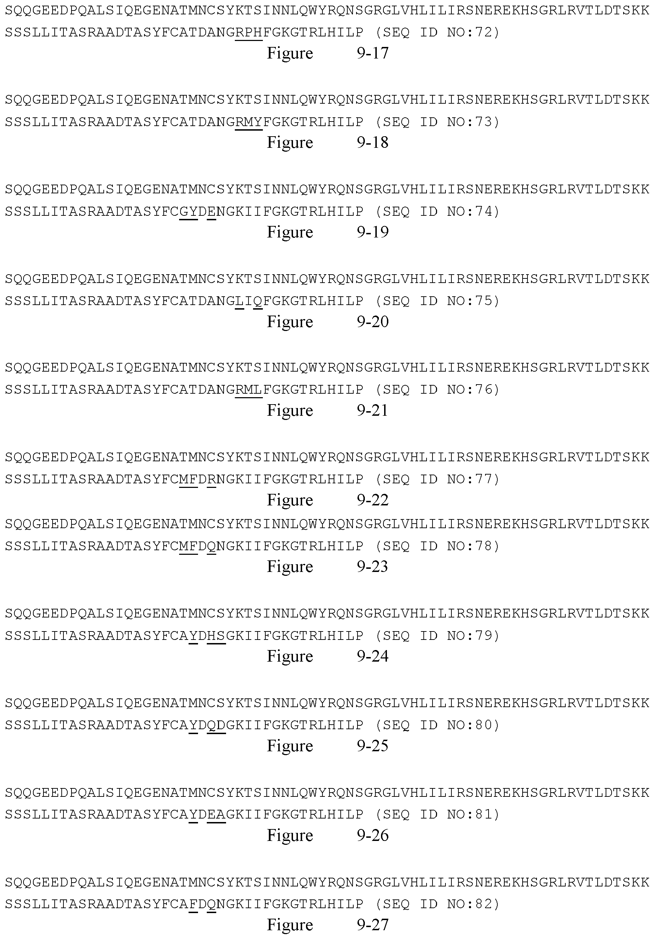

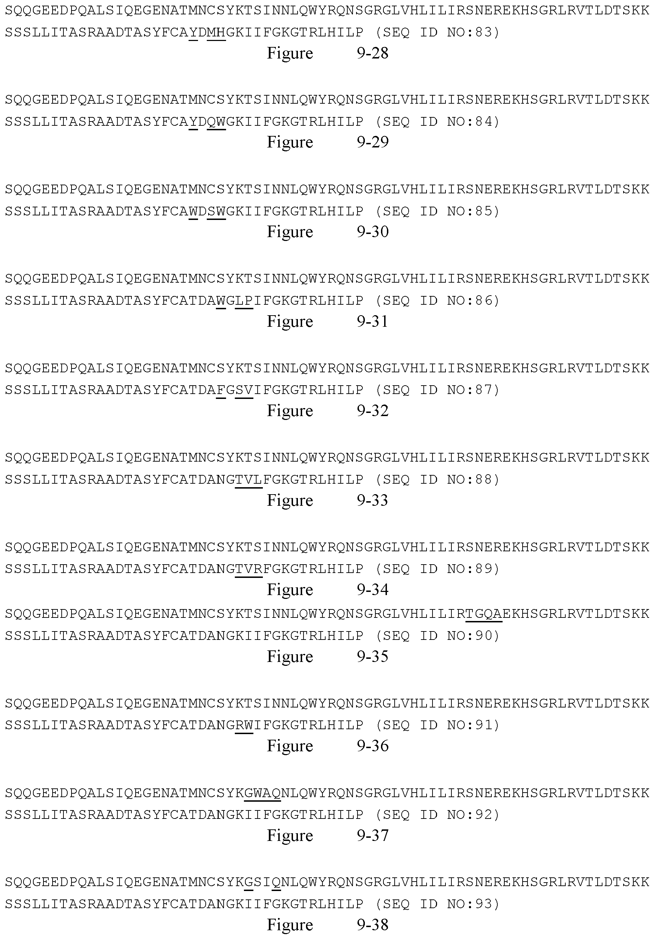

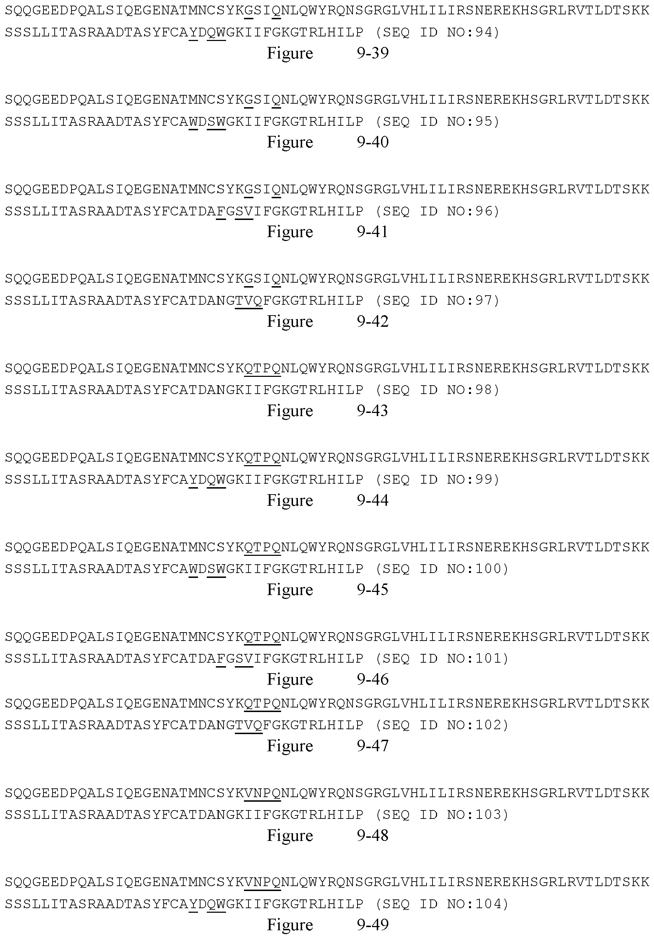

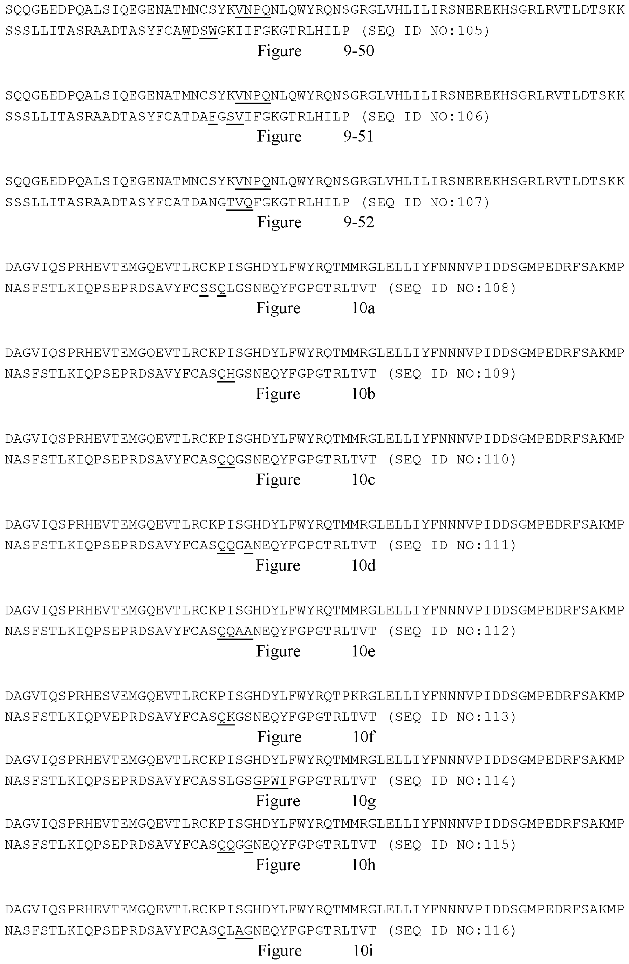

The CDRs selected from the α and β chains of the high affinity single chain TCR were transferred into the corresponding positions of the α chain variable domain SEQ ID NO: 1 and the β chain variable domain SEQ ID NO: 2 of the wild type TCR, respectively, to form an αβ heterodimeric TCR. Additionally, the αβ heterodimeric TCR is also formed through artificial combination of the screened mutation sites in CDRs. The amino acid sequences of α and β chain variable domains of the new TCR are shown in FIGS. 9-1 to 9-52 and FIGS. 10a to 10o, respectively. Since the CDRs of a TCR molecule determine the affinity of the TCR molecule for the corresponding pMHC complex, those skilled in the art can anticipate that the αβ heterodimeric TCR with high affinity mutation sites introduced also has high affinity for SLLMWITQC-HLA-A0201 complex. An expression vector was constructed by the method described in Example 5, and the preceding αβ heterodimeric TCR with high affinity mutations introduced was expressed, refolded and purified by the method described in Example 6. The affinity of the TCR for SLLMWITQC-HLA-A0201 complex was determined by BIAcore T200, as shown in Table 4.

**Table 4**

| TCR No. | TCR Variable Domain (SEQ ID NO) | | K_{D}(M) |
|---|---|---|---|
| | a | β | |
| 27 | 82 | 2 | 2.00E-06 |
| 22 | 77 | 2 | 1.68E-06 |
| 1 | 56 | 2 | 4.64E-07 |
| 2 | 57 | 2 | 6.07E-07 |
| 3 | 58 | 2 | 7.86E-07 |
| 4 | 59 | 2 | 4.83E-07 |
| 5 | 60 | 2 | 6.90E-07 |
| 6 | 61 | 2 | 2.13E-07 |
| 7 | 62 | 2 | 8.50E-07 |
| 8 | 63 | 2 | 1.13E-06 |
| 9 | 64 | 2 | 6.93E-07 |
| 10 | 65 | 2 | 1.08E-06 |
| 11 | 66 | 2 | 1.09E-06 |
| 12 | 67 | 2 | 1.04E-06 |
| 13 | 68 | 2 | 1.41E-06 |
| 14 | 69 | 2 | 2.41E-06 |
| 15 | 70 | 2 | 1.20E-06 |
| 16 | 71 | 2 | 5.02E-06 |
| 17 | 72 | 2 | 2.38E-06 |
| 18 | 73 | 2 | 3.11E-06 |
| 19 | 74 | 2 | 2.40E-06 |
| 20 | 75 | 2 | 3.77E-06 |
| 21 | 76 | 2 | 3.47E-06 |
| 23 | 78 | 2 | 1.21E-06 |
| 24 | 79 | 2 | 1.98E-06 |
| 25 | 80 | 2 | 1.58E-06 |
| 26 | 81 | 2 | 1.31E-06 |
| 28 | 83 | 2 | 5.62E-07 |
| 29 | 84 | 2 | 1.03E-08 |
| 30 | 85 | 2 | 7.57E-08 |
| 31 | 86 | 2 | 1.56E-08 |
| 32 | 87 | 2 | 1.96E-07 |
| 33 | 88 | 2 | 1.83E-07 |
| 34 | 89 | 2 | 3.40E-07 |
| 35 | 1 | 108 | 1.92E-07 |
| 36 | 1 | 109 | 1.90E-06 |
| 37 | 1 | 110 | 3.35E-06 |
| 38 | 1 | 111 | 1.40E-06 |
| 39 | 1 | 112 | 5.66E-06 |
| 40 | 1 | 113 | 4.74E-06 |
| 41 | 1 | 114 | 2.92E-06 |
| 42 | 1 | 115 | 7.75E-06 |
| 43 | 1 | 116 | 1.58E-05 |
| 44 | 90 | 2 | 2.22E-05 |
| 45 | 91 | 2 | 5.59E-07 |
| 46 | 1 | 117 | 4.11E-07 |
| 47 | 1 | 118 | 1.50E-06 |
| 48 | 85 | 117 | 6.79E-10 |
| 49 | 84 | 117 | 1.52E-10 |
| 50 | 85 | 119 | 1.39E-09 |
| 51 | 85 | 120 | 3.43E-09 |
| 52 | 92 | 2 | 2.66E-08 |
| 53 | 93 | 2 | 1.45E-07 |
| 54 | 94 | 117 | 1.4E-10 |
| 55 | 95 | 117 | 1.11E-10 |
| 56 | 96 | 117 | 1.14E-10 |
| 57 | 97 | 117 | 6.15E-11 |
| 58 | 94 | 121 | 3.76E-11 |
| 59 | 95 | 121 | 4.13E-11 |
| 60 | 96 | 121 | 1.61E-10 |
| 61 | 97 | 121 | 1.99E-10 |
| 62 | 94 | 122 | 8.70E-11 |
| 63 | 95 | 122 | 1.96E-10 |
| 64 | 96 | 122 | 2.62E-10 |
| 65 | 97 | 122 | 8.79E-11 |
| 66 | 98 | 2 | 1.09E-07 |
| 67 | 98 | 117 | 2.92E-09 |
| 68 | 98 | 119 | 3.22E-09 |
| 69 | 98 | 120 | 1.46E-08 |
| 70 | 99 | 117 | 1.18E-11 |
| 71 | 100 | 117 | 4.74E-11 |
| 72 | 101 | 117 | 3.13E-10 |
| 73 | 102 | 117 | 7.29E-11 |
| 74 | 99 | 121 | 6.06E-11 |
| 75 | 100 | 121 | 3.12E-11 |
| 76 | 101 | 121 | 1.72E-10 |
| 77 | 102 | 121 | 1.02E-10 |
| 78 | 99 | 122 | 2.12E-11 |
| 79 | 100 | 122 | 3.98E-11 |
| 80 | 101 | 122 | 2.09E-10 |
| 81 | 102 | 122 | 2.35E-10 |
| 82 | 103 | 2 | 8.76E-08 |
| 83 | 103 | 117 | 2.05E-09 |
| 84 | 103 | 119 | 3.20E-09 |
| 85 | 103 | 120 | 1.46E-08 |
| 86 | 104 | 117 | 1.92E-11 |
| 87 | 105 | 117 | 2.4E-11 |
| 88 | 106 | 117 | 1.14E-10 |
| 89 | 107 | 117 | 2.61E-10 |
| 90 | 104 | 121 | 2.24E-11 |
| 91 | 105 | 121 | 2.67E-11 |
| 92 | 106 | 121 | 1.32E-10 |
| 93 | 107 | 121 | 1.78E-10 |
| 94 | 104 | 122 | 3.81E-11 |
| 95 | 105 | 122 | 8.32E-11 |
| 96 | 106 | 122 | 4.47E-11 |
| 97 | 107 | 122 | 2.40E-10 |

As can be seen from Table 4, the obtained αβ heterodimeric TCR maintains high affinity for SLLMWITQC-HLA-A0201 complex. The affinity of the heterodimeric TCR for SLLMWITQC-HLA-A0201 complex is at least twice that of the wild type TCR.

### Example 8 Expression, refolding and purification of fusions of anti-CD3 antibodies with high affinity single chain TCRs

The high affinity single chain TCR molecule of the present disclosure was fused with a single-chain variable fragment (scFv) of an anti-CD3 antibody to construct a fusion molecule. Primers were designed by overlap PCR to ligate the genes of the anti-CD3 antibody and the high affinity single chain TCR molecule. An intermediate linker was designed as GGGGS, and the gene fragment of the fusion molecule had restriction enzyme sites NcoI and NotI. The PCR amplification product was digested with NcoI and NotI and ligated with pET28a vector digested with NcoI and NotI. The ligation product was transferred into *E. coli* DH5α competent cells, plated on a kanamycin-containing LB plate, inverted and cultured at 37 °C overnight, and the positive clones were picked for PCR screening. Positive recombinants were sequenced to determine the correct sequence and the recombinant plasmid was extracted and transferred into *E. coli* BL21 (DE3) competent cells for expression.

### Expression of fusion proteins

An expression plasmid containing target genes was transferred into *E. coli* strain BL21 (DE3), plated on an LB plate (kanamycin, 50 µg/mL) and cultured at 37 °C overnight. On the next day, the clones were picked and inoculated into 10 mL of LB liquid medium (kanamycin, 50 µg/mL), cultured for 2-3 h, then inoculated to 1 L of LB medium (kanamycin, 50 µg/mL) at a volume ratio of 1:100, cultured until OD₆₀₀ was 0.5-0.8, and then induced to express proteins of interest using IPTG with a final concentration of 0.5 mM. Four hours after induction, the cells were harvested by centrifugation at 6000 rpm for 10 min. The cells were washed once in a PBS buffer and dispensed. Cells corresponding to 200 mL of the bacterial culture were taken and lysed with 5 mL of BugBuster Master Mix (Novagen). The inclusion bodies were collected by centrifugation at 6000 g for 15 min. The inclusion bodies were washed 4 times with a detergent to remove cell debris and membrane components. The inclusion bodies were then washed with a buffer such as PBS to remove the detergent and salt. Finally, the inclusion bodies were dissolved in a Tris buffer solution containing 8 M urea, the concentration of the inclusion bodies was measured, and the inclusion bodies were dispensed and cryopreserved at -80 °C.

### Refolding of fusion proteins

About 10 mg of inclusion bodies were taken from a -80 °C ultra-low temperature freezer and thawed, added with dithiothreitol (DTT) to a final concentration of 10 mM and incubated at 37 °C for 30 min to 1 h to ensure complete opening of the disulfide bond. Then, the solution of inclusion body samples was added dropwise into 200 mL of 4 °C pre-cooled refolding buffer (100 mM Tris pH 8.1, 400 mM L-arginine, 2 mM EDTA, 5 M urea, 6.5 mM β-mercapthoethylamine, 1.87 mM cystamine), respectively and stirred slowly at 4 °C for about 30 min. The refolding solution was dialyzed against 8 volumes of pre-cooled H₂O for 16-20 h. It was further dialyzed twice against 8 volumes of 10 mM Tris pH 8.0, and the dialysis was continued at 4 °C for about 8 h. After dialysis, the sample was filtered and subjected to the following purification process.

### First step purification of fusion proteins

The dialyzed and refolded material (in 10 mM Tris pH 8.0) was eluted on a POROS HQ/20 anion exchange chromatography prepacked column (Applied Biosystems) with a gradient of 0-600 mM NaCl using AKTA Purifier (GE Healthcare). Each component was analyzed by Coomassie brilliant blue-stained SDS -PAGE and then combined.

### Second step purification of fusion proteins

The purified and combined sample solution in the first step was concentrated for purification in this step. The fusion protein was purified by Superdex 75 10/300 GL gel permeation chromatography prepacked column (GE Healthcare) pre-equilibrated in the PBS buffer. The components of the peaks were analyzed by Coomassie brilliant blue-stained SDS-PAGE and then combined.

### Example 9 Expression, refolding and purification of fusions of anti-CD3 antibodies with αβ heterodimeric TCRs with high affinity

An anti-CD3 single-chain antibody (scFv) was fused with an αβ heterodimeric TCRA to prepare a fusion molecule. The anti-CD3 scFv was fused with the β chain of the TCR, where the β chain of the TCR may comprise the β chain variable domain of any one of the above αβ heterodimeric TCRs with high affinity, and the TCR α chain of the fusion molecule may comprise the α chain variable domain of any one of the above αβ heterodimeric TCRs with high affinity.

### Construction of an expression vector for the fusion molecule

### 1. Construction of an expression vector for the α chain

The target gene carrying the α chain of the αβ heterodimeric TCR was digested with NcoI and NotI and ligated with pET28a vector digested with NcoI and NotI. The ligation product was transferred into *E. coli* DH5α, plated on a kanamycin-containing LB plate, inverted and cultured at 37 °C overnight, and the positive clones were picked for PCR screening. Positive recombinants were sequenced to determine the correct sequence and the recombinant plasmid was extracted and transferred into *E. coli* Tuner (DE3) for expression.

### 2. Construction of an expression vector for anti-CD3 (scFv)-β chain

Primers were designed by overlap PCR to ligate the genes of the anti-CD3 scFv and the β chain of the heterodimeric TCR with high affinity. An intermediate linker was GGGGS, and the gene fragment of the fusion protein of the anti-CD3 scFv and the β chain of the heterodimeric TCR with high affinity had restriction enzyme sites NcoI (CCATGG) and NotI (GCGGCCGC). The PCR amplification product was digested with NcoI and NotI and ligated with pET28a vector digested with NcoI and NotI. The ligation product was transferred into *E. coli* DH5α competent cells, plated on a kanamycin-containing LB plate, inverted and cultured at 37 °C overnight, and the positive clones were picked for PCR screening. Positive recombinants were sequenced to determine the correct sequence and the recombinant plasmid was extracted and transferred into *E. coli* Tuner (DE3) competent cells for expression.

### Expression, refolding and purification of fusion proteins

The expression plasmids were separately transferred into *E. coli* Tuner (DE3) competent cells, plated on an LB plate (kanamycin, 50 µg/mL) and cultured overnight at 37 °C. On the next day, the clones were picked and inoculated into 10 mL of LB liquid medium (kanamycin, 50 µg/mL), cultured for 2-3 h, then inoculated to 1 L of LB medium at a volume ratio of 1:100, cultured until OD₆₀₀ was 0.5-0.8, and then induced to express proteins of interest using IPTG with a final concentration of 1 mM. Four hours after induction, the cells were harvested by centrifugation at 6000 rpm for 10 min. The cells were washed once in a PBS buffer and dispensed. Cells corresponding to 200 mL of the bacterial culture were taken and lysed with 5 mL of BugBuster Master Mix (Merck). The inclusion bodies were collected by centrifugation at 6000 g for 15 min. The inclusion bodies were washed 4 times with a detergent to remove cell debris and membrane components. The inclusion bodies were then washed with a buffer such as PBS to remove the detergent and salt. Finally, the inclusion bodies were dissolved in a buffer solution of 6M guanidine hydrochloride, 10 mM dithiothreitol (DTT), 10 mM ethylenediaminetetraacetic acid (EDTA), 20 mM Tris, pH 8.1, and the concentration of the inclusion bodies was determined. The inclusion bodies were dispensed and cryopreserved at-80 °C.

The dissolved TCR α chain and anti-CD3 (scFv)-β chain were rapidly mixed in a mass ratio of 2:5 in 5 M urea, 0.4 M L-arginine, 20 mM Tris pH 8.1, 3.7 mM cystamine and 6.6 mM β-mercapoethylamine (4 °C), and the final concentrations of the α chain and the anti-CD3 (scFv)-β chain were 0.1 mg/mL and 0.25 mg/mL, respectively.

After mixing, the solution was dialyzed against 10 volumes of deionized water (4 °C). After 12 h, deionized water was replaced with a buffer (10 mM Tris, pH 8.0) and dialysis was continued at 4 °C for 12 h. After completion of the dialysis, the solution was filtered through a 0.45 µM filter and purified through an anion exchange column (HiTrap Q HP, 5 mL, GE Healthcare). The elution peak of the TCR containing the successfully refolded TCR α chain and anti-CD3 (scFv)-β chain dimer was confirmed by SDS-PAGE gel. The TCR fusion molecule was then purified by size-exclusion chromatography (S-100 16/60, GE Healthcare) and further purified by an anion exchange column (HiTrap Q HP, 5 mL, GE healthcare). The purity of the purified TCR fusion molecule was determined by SDS-PAGE to be greater than 90%, and the concentration thereof was determined by the BCA method.

### Example 10 Activation function assay of effector cells transfected with the high affinity TCR of the present disclosure (with a tumor cell line as target cells)

This example demonstrates that effector cells transfected with the high affinity TCR of the present disclosure have a good specific activation effect on target cells. The function and specificity of the high affinity TCR of the present disclosure in cells were detected by an ELISPOT assay.

Methods for detecting cellular functions using the ELISPOT assay are well known to those skilled in the art. PBLs isolated from the blood of healthy volunteers were transfected with the randomly selected TCRs of the present disclosure as effector cells. The TCRs and their numbers are known from Table 4, which are respectively TCR22 (α chain variable domain SEQ ID NO: 77 and β chain variable domain SEQ ID NO: 2), TCR23 (α chain variable domain SEQ ID NO: 78 and β chain variable domain SEQ ID NO: 2), TCR24 (α chain variable domain SEQ ID NO: 79 and β chain variable domain SEQ ID NO: 2), TCR25 (α chain variable domain SEQ ID NO: 80 and β chain variable domain SEQ ID NO: 2), TCR26 (α chain variable domain SEQ ID NO: 81 and β chain variable domain SEQ ID NO: 2), TCR27 (α chain variable domain SEQ ID NO: 82 and β chain variable domain SEQ ID NO: 2) and TCR28 (α chain variable domain SEQ ID NO: 83 and β chain variable domain SEQ ID NO: 2). Effector cells in control groups were labeled as wild type TCR (cells transfected with a wild type TCR) and A6 (cells transfected with another TCR). The target cell lines were A375, Mel526, NCI-H1650 and NCI-H1299 cells. The target cell line A375 expresses relevant antigens and was used as a positive tumor cell line; Mel526 and NCI-H1650 express no relevant antigens, and NCI-H1299 is a non-HLA-A0201 cell line, and were used as negative tumor cell lines as control.

Firstly, an ELISPOT plate was prepared. The ELISPOT plate was activated and coated with ethanol overnight at 4 °C. On the first day of the assay, the coating solution was removed, the plate was washed, blocked and incubated at room temperature for 2 h, and the blocking solution was removed. Components of the assay were added to the ELISPOT plate: 20000 target cells/well, 1000 effector cells/well (calculated according to the positive rate of transfection), and two duplicate wells were set. The ELISPOT plate was incubated overnight (37 °C, 5% CO₂). On the second day of the assay, the plate was washed, subjected to secondary detection and development and dried, and the spots formed on the film were counted using an immunospot plate reader (ELISPOT READER system; AID20 Company).

The results of the assay are shown in FIG. 13. The effector cells transfected with the high affinity TCR of the present disclosure exhibit a good specific activation effect on target cells and have a far better function than the effector cells transfected with the wild type TCR.

### Example 11 Activation function assay of effector cells transfected with the high affinity TCR of the present disclosure (with T2 loaded with related short peptides as target cells)

This example demonstrates that effector cells transfected with the high affinity TCR of the present disclosure have a good specific activation effect on artificially prepared target cells from another aspect. Methods for detecting cellular activation functions using an ELISPOT assay are well known to those skilled in the art. CD8⁺ T cells isolated from the blood of healthy volunteers were transfected with the randomly selected TCRs of the present disclosure as effector cells. The TCRs and their numbers are known from Table 4, which are respectively TCR22 (α chain variable domain SEQ ID NO: 77 and β chain variable domain SEQ ID NO: 2), TCR23 (α chain variable domain SEQ ID NO: 78 and β chain variable domain SEQ ID NO: 2), TCR24 (α chain variable domain SEQ ID NO: 79 and β chain variable domain SEQ ID NO: 2), TCR25 (α chain variable domain SEQ ID NO: 80 and β chain variable domain SEQ ID NO: 2), TCR26 (α chain variable domain SEQ ID NO: 81 and β chain variable domain SEQ ID NO: 2), TCR27 (α chain variable domain SEQ ID NO: 82 and β chain variable domain SEQ ID NO: 2) and TCR28 (α chain variable domain SEQ ID NO: 83 and β chain variable domain SEQ ID NO: 2). Effector cells in control groups were labeled as wild type TCR (cells transfected with a wild type TCR) and A6 (cells transfected with another TCR). In this example, the target cells are T2 cells loaded with specific short peptides.

Firstly, an ELISPOT plate was prepared. The ELISPOT plate was activated and coated with ethanol overnight at 4 °C. On the first day of the assay, the coating solution was removed, the plate was washed, blocked and incubated at room temperature for 2 h, and the blocking solution was removed. Components of the assay were added to the ELISPOT plate: 20000 target cells/well, 1000 effector cells/well (calculated according to the positive rate of transfection), and the final concentrations of the short peptides in the wells of the ELISPOT plate are at eight gradients of 1 × 10⁻¹³g/mL to 1 × 10⁻⁶g/mL. Two duplicate wells were set. The ELISPOT plate was incubated overnight (37 °C, 5% CO₂). On the second day of the assay, the plate was washed, subjected to secondary detection and development and dried, and the spots formed on the film were counted using an immunospot plate reader (ELISPOT READER system; AID20 Company).

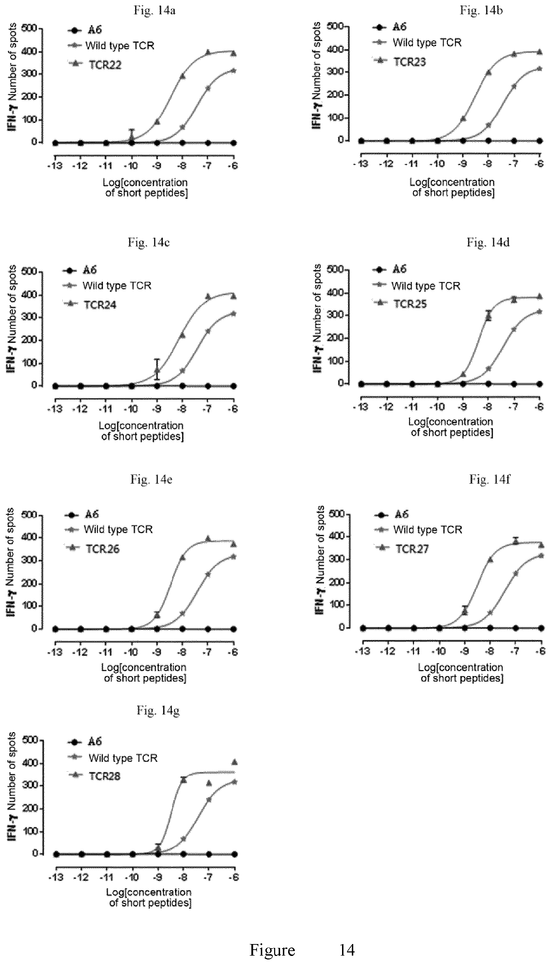

The results of the assay are shown in FIG. 14. The effector cells transfected with the TCR of the present disclosure exhibit a strong activation effect on the target cells loaded with specific short peptides and have a far better function than the effector cells transfected with the wild type TCR, while the effector cells transfected with another TCR have no activation effect on the target cells.

### Example 12 ELISA IL-2 activation function assay of effector cells transfected with the high affinity TCR of the present disclosure

This example verifies the activation function of the cells transduced with the TCR of the present disclosure by measuring the release of IL-2 through ELISA.

Methods for detecting cellular functions using the release of IL-2 are well known to those skilled in the art. PBLs isolated from the blood of healthy volunteers were transfected with the randomly selected TCRs of the present disclosure as effector cells. The TCRs and their numbers are known from Table 4, which are respectively TCR22 (α chain variable domain SEQ ID NO: 77 and β chain variable domain SEQ ID NO: 2), TCR23 (α chain variable domain SEQ ID NO: 78 and β chain variable domain SEQ ID NO: 2), TCR24 (α chain variable domain SEQ ID NO: 79 and β chain variable domain SEQ ID NO: 2), TCR25 (α chain variable domain SEQ ID NO: 80 and β chain variable domain SEQ ID NO: 2) and TCR27 (α chain variable domain SEQ ID NO: 82 and β chain variable domain SEQ ID NO: 2). Effector cells in control groups were labeled as wild type TCR (cells transfected with a wild type TCR) and A6 (cells transfected with another TCR). The target cell lines were A375, Mel526, NCI-H1650 and NCI-H1299 cells. A375 expresses NY-ESO-1 antigen and was used as a positive tumor cell line; NCI-H1650 and MEL526 basically do not express NY-ESO-1 antigen, and NCI-H1299 is a non-HLA-A0201 cell, and were used as negative tumor cell lines as control.

Firstly, a 96-well plate was prepared. On the first day of the assay, components of the assay were added to the plate: 100000 target cells/well, 100000 effector cells/well (calculated according to the positive rate of transfection), and three duplicate wells were set. The plate was incubated overnight (37 °C, 5% CO₂). On the second day of the assay, the supernatant was seeded into an ELISA plate and subjected to detection and development according to the instructions of an ELISA kit. After the reaction was terminated, the absorbance was recorded at 450 nm with a microplate reader (Bioteck).

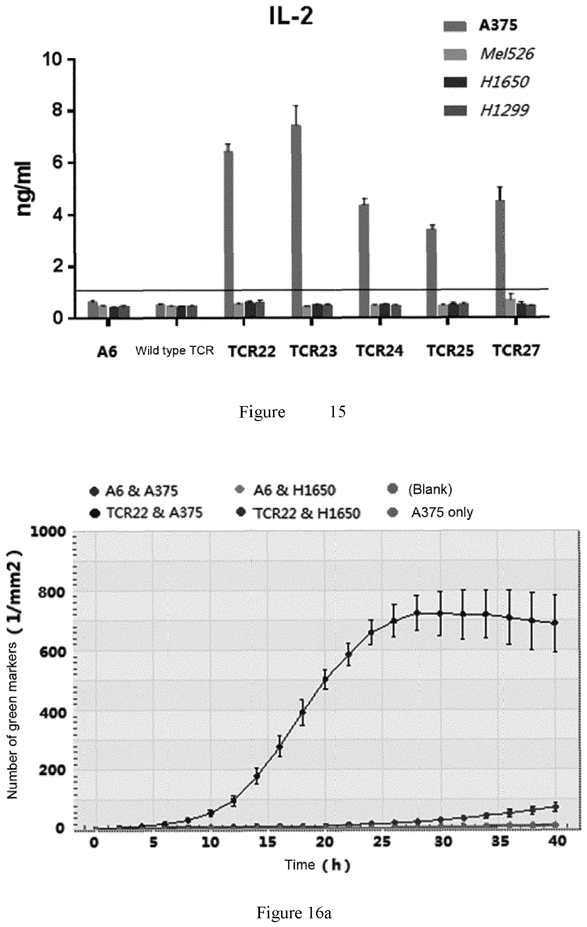

The results of the assay are shown in FIG. 15. The cells transduced with the TCR of the present disclosure have a strong activation effect on the positive tumor cell line and almost have no activation effect on the negative tumor cell line.

### Example 13 IncuCyte killing function assay of effector cells transfected with the high affinity TCR of the present disclosure

This example demonstrates that effector cells transfected with the high affinity TCR of the present disclosure have a good specific killing effect on target cells.

A method for detecting cellular functions using an IncuCyte assay is well known to those skilled in the art, which is a non-invasive method to record the real-time growth state of cells. PBLs isolated from the blood of healthy volunteers were transfected with the randomly selected TCRs of the present disclosure as effector cells. The TCRs and their numbers are known from Table 4, which are respectively TCR22 (α chain variable domain SEQ ID NO: 77 and β chain variable domain SEQ ID NO: 2), TCR23 (α chain variable domain SEQ ID NO: 78 and β chain variable domain SEQ ID NO: 2), TCR24 (α chain variable domain SEQ ID NO: 79 and β chain variable domain SEQ ID NO: 2), TCR25 (α chain variable domain SEQ ID NO: 80 and β chain variable domain SEQ ID NO: 2) and TCR27 (α chain variable domain SEQ ID NO: 82 and β chain variable domain SEQ ID NO: 2). Effector cells in control groups were labeled as wild type TCR (cells transfected with a wild type TCR) and A6 (cells transfected with another TCR). The target cell lines were A375 and NCI-H1650 cells. The target cell line A375 expresses relevant antigens; and NCI-H1650 expresses no relevant antigens and was used as a control.

On the first day of the assay, the target cells were digested, centrifuged, resuspended in a complete medium of RPMI1640+10% FBS without phenol red, counted and with 1^{∗}10⁴ cells/well/100uL, evenly spread on a 96-well plate; the plate was put back in a 37 °C, 5% CO₂ incubator and incubated overnight. On the second day, the medium in the 96-well plate was discarded and replaced with a phenol red-free RPMI1640+10%FBS medium containing dye caspase3/7 reagent, where the concentration of the dye was 2 drops/mL. The old medium was discarded and replaced with a new phenol red-free RPMI1640+10% FBS medium. Effector cells at 1^{∗}10⁴ cells/well/100uL (calculated according to the positive rate of transfection) and an experimental group plated with target cells were co-incubated. The plate was incubated for half an hour in a real-time dynamic live cell imaging analyzer IncuCyte ZooM dedicated for IncuCyte detection and observed and photographed in real time. The detection results were processed and the data was analyzed and exported using IncuCyte ZooM 2016A.

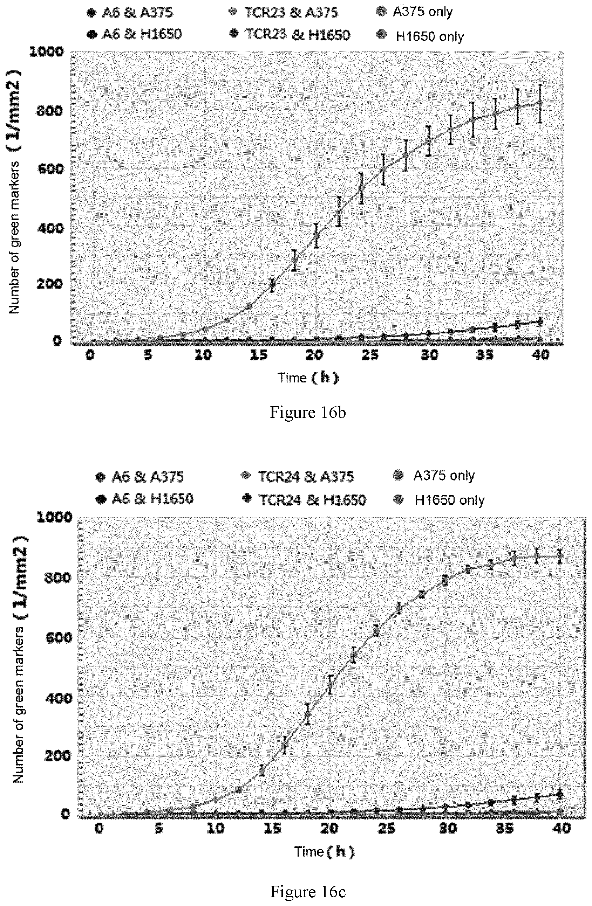

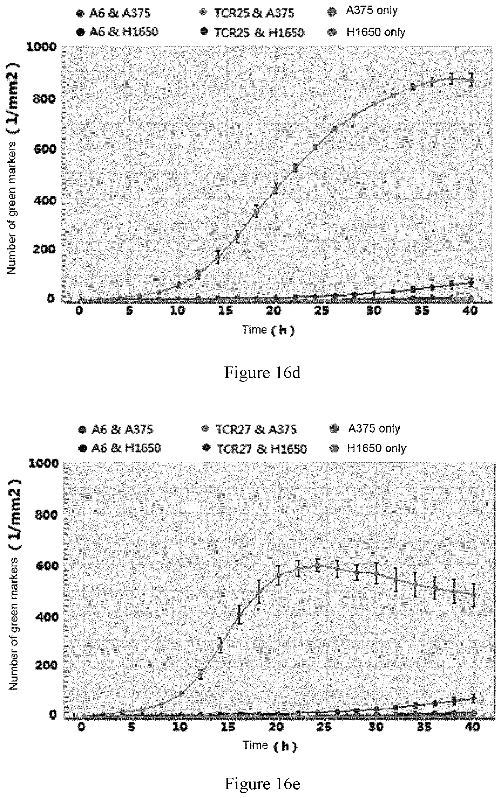

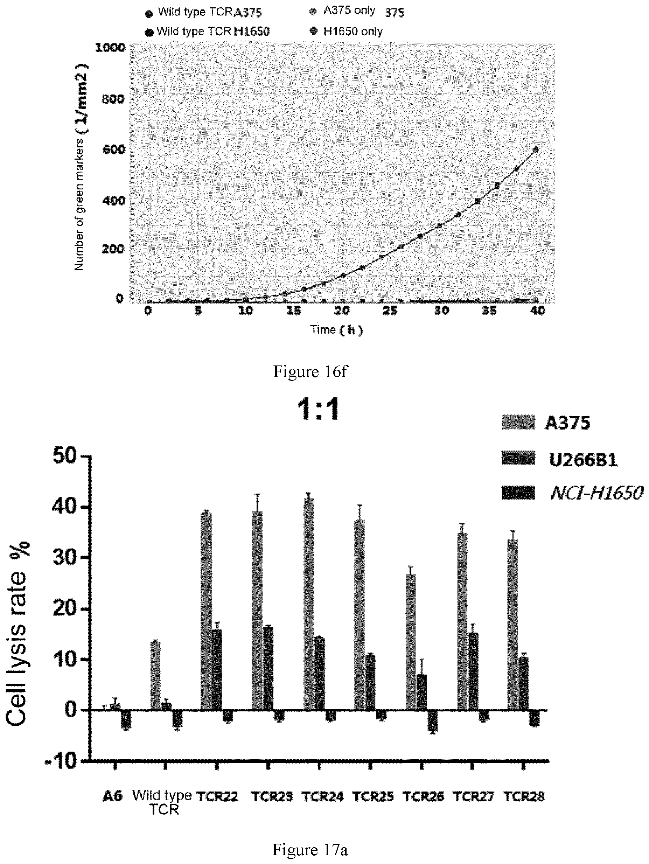

As shown by the detection results in FIGS. 16a to 16f, compared with those transduced with the wild type TCR, the cells transduced with the TCR of the present disclosure kill the positive tumor cell line significantly faster and have a significantly enhanced killing effect while the effector cells transfected with another TCR almost have no killing effect.

### Example 14 LDH killing function assay of effector cells transfected with the high affinity TCR of the present disclosure

This example verifies the killing function of the cells transduced with the TCR of the present disclosure by measuring the release of LDH through a non-radioactive cytotoxicity assay. The assay is a colorimetric alternative to the 51Cr release cytotoxicity assay and quantifies lactate dehydrogenase (LDH) released after cell lysis. The LDH released in a culture medium was detected using a 30-minute coupled enzymatic reaction where the LDH converts a tetrazolium salt (INT) to red formazan. The amount of the red product produced is proportional to the number of cells lysed. Visible absorbance data at 490 nm can be collected using a standard 96-well plate reader.

Methods for detecting cellular functions using the release of LDH are well known to those skilled in the art. PBLs isolated from the blood of healthy volunteers were transfected with the randomly selected TCRs of the present disclosure as effector cells. The TCRs and their numbers are known from Table 4, which are respectively TCR22 (α chain variable domain SEQ ID NO: 77 and β chain variable domain SEQ ID NO: 2), TCR23 (α chain variable domain SEQ ID NO: 78 and β chain variable domain SEQ ID NO: 2), TCR24 (α chain variable domain SEQ ID NO: 79 and β chain variable domain SEQ ID NO: 2), TCR25 (α chain variable domain SEQ ID NO: 80 and β chain variable domain SEQ ID NO: 2), TCR26 (α chain variable domain SEQ ID NO: 81 and β chain variable domain SEQ ID NO: 2), TCR27 (α chain variable domain SEQ ID NO: 82 and β chain variable domain SEQ ID NO: 2) and TCR28 (α chain variable domain SEQ ID NO: 83 and β chain variable domain SEQ ID NO: 2). Effector cells in control groups were labeled as wild type TCR (cells transfected with a wild type TCR) and A6 (cells transfected with another TCR). The target cell lines were A375, U266B1 and NCI-H1650 cells. A375 and U266B1 express NY-ESO-1 antigen; NCI-H1650 basically does not express NY-ESO-1 antigen as a control.

Firstly, an LDH plate was prepared. On the first day of the assay, components of the assay were added to the plate: 50000 effector cells/well and 50000 target cells/well were added at 1:1 into the corresponding wells or 150000 effector cells/well and 50000 target cells/well were added at 3:1 to the corresponding wells to form two experimental groups, and three duplicate wells were set. The plate was incubated overnight (37 °C, 5% CO₂). On the second day of the assay, the cells were subjected to detection and development. After the reaction was terminated, the absorbance was recorded at 450 nm with a microplate reader (Bioteck).

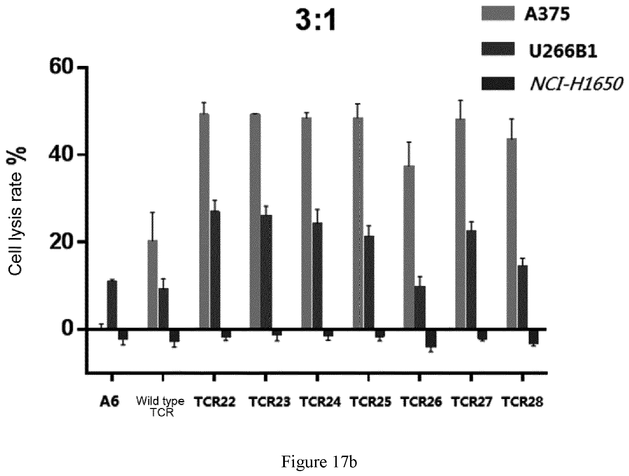

The results of the assay are shown in FIGS. 17a and 17b. The cells transduced with the TCR of the present disclosure have a very strong killing effect on the target cells expressing the relevant antigens in the two experimental groups with different effector-target ratios of 1:1 and 3:1 and almost have no killing effect on the target cells that do not express the relevant antigens.

### Example 15 Function assay of fusion proteins of the high affinity TCR of the present disclosure and an anti-CD3 antibody

This example demonstrates that the fusion proteins of the high affinity TCR of the present disclosure and the anti-CD3 antibody can redirect effector cells and exhibit a good activation effect.

Methods for detecting cellular functions using an ELISPOT assay are well known to those skilled in the art. The effector cells used in IFN-γ ELISPOT assay of this example were CD8⁺ T cells isolated from the blood of healthy volunteers. The target cell lines were A375-A2, Mel526, NCI-H1650 and NCI-H1299. A375-A2 expresses relevant antigens, Mel526 and NCI-H1650 do not express relevant antigens, and NCI-H1299 is a non-HLA-A0201 cell line, and were used as control. The fusion proteins were prepared as described in Example 8 from the randomly selected high affinity TCRs of the present disclosure, which are respectively named as fusion protein 1 (α chain SEQ ID NO: 94 and β chain SEQ ID NO: 117), fusion protein 2 (α chain SEQ ID NO: 96 and β chain SEQ ID NO: 117), fusion protein 3 (α chain SEQ ID NO: 94 and β chain SEQ ID NO: 122), fusion protein 4 (α chain SEQ ID NO: 100 and β chain SEQ ID NO: 117), fusion protein 5 (α chain SEQ ID NO: 100 and β chain SEQ ID NO: 122), fusion protein 6 (α chain SEQ ID NO: 101 and β chain SEQ ID NO: 122), fusion protein 7 (α chain SEQ ID NO: 106 and β chain SEQ ID NO: 117), fusion protein 8 (α chain SEQ ID NO: 107 and β chain SEQ ID NO: 117), fusion protein 9 (α chain SEQ ID NO: 106 and β chain SEQ ID NO: 122) and fusion protein 10 (α chain SEQ ID NO: 107 and β chain SEQ ID NO: 122).

Firstly, an ELISPOT plate was prepared. The ELISPOT plate was activated and coated with ethanol overnight at 4 °C. On the first day of the assay, the coating solution was removed, the plate was washed, blocked and incubated at room temperature for 2 h, and the blocking solution was removed. Components of the assay were added to the ELISPOT plate: the fusion protein dilution, 10000 target cells/well, 2000 effector cells/well were added into the corresponding wells and the final concentrations of the fusion proteins in the wells of the ELISPOT plate were at seven gradients ranging from 1 × 10⁻¹⁴g/mL to 1 × 10⁻⁸g/mL. Two duplicate wells were set. The plate was incubated overnight (37 °C, 5% CO₂). On the second day of the assay, the plate was washed, subjected to secondary detection and development and dried, and the spots formed on the film were counted using an immunospot plate reader (ELISPOT READER system; AID20 Company).

The results of the assay are shown in FIGS. 8a to 18j. The fusion proteins of the high affinity TCR of the present disclosure and the anti-CD3 antibody can redirect effector cells and exhibit a good activation effect.

All documents mentioned in the present disclosure are hereby incorporated by reference in their entireties as if each is incorporated by reference. In addition, it is to be understood that after reading the teachings of the present disclosure, those skilled in the art can make various changes or modifications to the present disclosure, and these equivalent forms also fall within the scope as defined by the appended claims of the present application.

## Claims

1. A T-cell receptor (TCR), wherein the TCR has an activity of binding to SLLMWITQC-HLA A0201 complex, an α chain variable domain of the TCR comprises an amino acid sequence having at least 90% of sequence homology with the amino acid sequence as shown in SEQ ID NO: 1; and a β chain variable domain of the TCR comprises an amino acid sequence having at least 90% of sequence homology with the amino acid sequence as shown in SEQ ID NO: 2.

2. The TCR of claim 1, wherein the affinity of the TCR for SLLMWITQC-HLA A0201 complex is at least twice that of a wild type TCR.

3. The TCR of claim 1, wherein the TCR comprises a TCR α chain variable domain and a TCR β chain variable domain, the TCR α chain variable domain comprises CDR1α, CDR2α and CDR3α, and the TCR β chain variable domain comprises CDR1β, CDR2β and CDR3β, wherein the amino acid sequence of CDR1β is SGHDY.

4. The TCR of claim 3, wherein the amino acid sequence of CDR2α is IRSNERE.

5. The TCR of claim 3, wherein the amino acid sequence of CDR1α is selected from TSINN, QTPQN, VNPQN and GSIQN.

6. The TCR of claim 3, wherein the amino acid sequence of CDR2β is selected from FNNNVP and FNHGAL.

7. The TCR of claim 3, wherein the amino acid sequence of CDR3β is selected from ASQLGSNEQY and ASQLGPNEQY

8. The TCR of claim 3, wherein the TCR has an activity of binding to SLLMWITQC-HLA A0201 complex and comprises a TCR α chain variable domain and a TCR β chain variable domain; wherein the TCR contains a mutation in the α chain variable domain shown in SEQ ID NO: 1, and the mutation is selected from one or more of T27G/Q/V, S28W/T/N, I29A/P, N30Q, S51T, N52G, E53Q, R54A, A90G/L/M, T91F/W/Y, A93E/H/M/Q/R/S, N94A/D/F/H/S/W, G95A, K96R/Q/S/L/T/W, I97W/V/M/P and I98F/E/D/H/L/Q/R/T/Y, wherein amino acid residues are numbered as shown in SEQ ID NO: 1; and the TCR contains a mutation in the β chain variable domain shown in SEQ ID NO: 2, and the mutation is selected from one or more of N51H, N52G, V53A, P54L, A93S, S95Q, L96R/K/H/Q, G97A, S98A/G/P, N99G, E100P, Q101W and Y102I, wherein amino acid residues are numbered as shown in SEQ ID NO: 2.

9. The TCR of claim 1, wherein the TCR has CDRs selected from the group consisting of:
| CDR No. | CDR1α | CDR2a | CDR3α | CDR1β | CDR2β | CDR3β |
|---|---|---|---|---|---|---|
| 27 | TSINN | IRSNERE | AFDQNGKII | SGHDY | FNNNVP | ASSLGSNEQY |
| 22 | TSINN | IRSNERE | MFDRNGKII | SGHDY | FNNNVP | ASSLGSNEQY |
| 1 | TSINN | IRSNERE | ATDANGSIF | SGHDY | FNNNVP | ASSLGSNEQY |
| 2 | TSINN | IRSNERE | ATDANGRWR | SGHDY | FNNNVP | ASSLGSNEQY |
| 3 | TSINN | IRSNERE | ATDANGWVQ | SGHDY | FNNNVP | ASSLGSNEQY |
| 4 | TSINN | IRSNERE | ATDANGWMQ | SGHDY | FNNNVP | ASSLGSNEQY |
| 5 | TSINN | IRSNERE | ATDANGLPH | SGHDY | FNNNVP | ASSLGSNEQY |
| 6 | TSINN | IRSNERE | ATDANGTIE | SGHDY | FNNNVP | ASSLGSNEQY |
| 7 | TSINN | IRSNERE | ATDANGLPQ | SGHDY | FNNNVP | ASSLGSNEQY |
| 8 | TSINN | IRSNERE | ATDANGLPE | SGHDY | FNNNVP | ASSLGSNEQY |
| 9 | TSINN | IRSNERE | ATDANGSMQ | SGHDY | FNNNVP | ASSLGSNEQY |
| 10 | TSINN | IRSNERE | ATDANGLPT | SGHDY | FNNNVP | ASSLGSNEQY |
| 11 | TSINN | IRSNERE | ATDAQGRWI | SGHDY | FNNNVP | ASSLGSNEQY |
| 12 | TSINN | IRSNERE | ATDANGSID | SGHDY | FNNNVP | ASSLGSNEQY |
| 13 | TSINN | IRSNERE | AYDQNGKII | SGHDY | FNNNVP | ASSLGSNEQY |
| 14 | TSINN | IRSNERE | ATDANGQPR | SGHDY | FNNNVP | ASSLGSNEQY |
| 15 | TSINN | IRSNERE | LYDQNGKII | SGHDY | FNNNVP | ASSLGSNEQY |
| 16 | TSINN | IRSNERE | ATDANAQVR | SGHDY | FNNNVP | ASSLGSNEQY |
| 17 | TSINN | IRSNERE | ATDANGRPH | SGHDY | FNNNVP | ASSLGSNEQY |
| 18 | TSINN | IRSNERE | ATDANGRMY | SGHDY | FNNNVP | ASSLGSNEQY |
| 19 | TSINN | IRSNERE | GYDENGKII | SGHDY | FNNNVP | ASSLGSNEQY |
| 20 | TSINN | IRSNERE | ATDANGLIQ | SGHDY | FNNNVP | ASSLGSNEQY |
| 21 | TSINN | IRSNERE | ATDANGRML | SGHDY | FNNNVP | ASSLGSNEQY |
| 23 | TSINN | IRSNERE | MFDQNGKII | SGHDY | FNNNVP | ASSLGSNEQY |
| 24 | TSINN | IRSNERE | AYDHSGKII | SGHDY | FNNNVP | ASSLGSNEQY |
| 25 | TSINN | IRSNERE | AYDQDGKII | SGHDY | FNNNVP | ASSLGSNEQY |
| 26 | TSINN | IRSNERE | AYDEAGKII | SGHDY | FNNNVP | ASSLGSNEQY |
| 28 | TSINN | IRSNERE | AYDMHGKII | SGHDY | FNNNVP | ASSLGSNEQY |
| 29 | TSINN | IRSNERE | AYDQWGKII | SGHDY | FNNNVP | ASSLGSNEQY |
| 30 | TSINN | IRSNERE | AWDSWGKII | SGHDY | FNNNVP | ASSLGSNEQY |
| 31 | TSINN | IRSNERE | ATDAWGLPI | SGHDY | FNNNVP | ASSLGSNEQY |
| 32 | TSINN | IRSNERE | ATDAFGSVI | SGHDY | FNNNVP | ASSLGSNEQY |
| 33 | TSINN | IRSNERE | ATDANGTVL | SGHDY | FNNNVP | ASSLGSNEQY |
| 34 | TSINN | IRSNERE | ATDANGTVR | SGHDY | FNNNVP | ASSLGSNEQY |
| 35 | TSINN | IRSNERE | ATDANGKII | SGHDY | FNNNVP | SSQLGSNEQY |
| 36 | TSINN | IRSNERE | ATDANGKII | SGHDY | FNNNVP | ASQHGSNEQY |
| 37 | TSINN | IRSNERE | ATDANGKII | SGHDY | FNNNVP | ASQQGSNEQY |
| 38 | TSINN | IRSNERE | ATDANGKII | SGHDY | FNNNVP | ASQQGANEQY |
| 39 | TSINN | IRSNERE | ATDANGKII | SGHDY | FNNNVP | ASQQAANEQY |
| 40 | TSINN | IRSNERE | ATDANGKII | SGHDY | FNNNVP | ASQKGSNEQY |
| 41 | TSINN | IRSNERE | ATDANGKII | SGHDY | FNNNVP | ASSLGSGPWI |
| 42 | TSINN | IRSNERE | ATDANGKII | SGHDY | FNNNVP | ASQQGGNEQY |
| 43 | TSINN | IRSNERE | ATDANGKII | SGHDY | FNNNVP | ASQLAGNEQY |
| 44 | TSINN | IRTGQAE | ATDANGKII | SGHDY | FNNNVP | ASSLGSNEQY |
| 45 | TSINN | IRSNERE | ATDANGRWI | SGHDY | FNNNVP | ASSLGSNEQY |
| 46 | TSINN | IRSNERE | ATDANGKII | SGHDY | FNHGAL | ASSLGSNEQY |
| 47 | TSINN | IRSNERE | ATDANGKII | SGHDY | FNNNVP | ASQRGSNEQY |
| 48 | TSINN | IRSNERE | AWDSWGKII | SGHDY | FNHGAL | ASSLGSNEQY |
| 49 | TSINN | IRSNERE | AYDQWGKII | SGHDY | FNHGAL | ASSLGSNEQY |
| 50 | TSINN | IRSNERE | AWDSWGKII | SGHDY | FNNNVP | ASQLGSNEQY |
| 51 | TSINN | IRSNERE | AWDSWGKII | SGHDY | FNNNVP | ASQLGPNEQY |
| 52 | GWAQN | IRSNERE | ATDANGKII | SGHDY | FNNNVP | ASSLGSNEQY |
| 53 | GSIQN | IRSNERE | ATDANGKII | SGHDY | FNNNVP | ASSLGSNEQY |
| 54 | GSIQN | IRSNERE | AYDQWGKII | SGHDY | FNHGAL | ASSLGSNEQY |
| 55 | GSIQN | IRSNERE | AWDSWGKII | SGHDY | FNHGAL | ASSLGSNEQY |
| 56 | GSIQN | IRSNERE | ATDAFGSVI | SGHDY | FNHGAL | ASSLGSNEQY |
| 57 | GSIQN | IRSNERE | ATDANGTVQ | SGHDY | FNHGAL | ASSLGSNEQY |
| 58 | GSIQN | IRSNERE | AYDQWGKII | SGHDY | FNHGAL | ASQLGSNEQY |
| 59 | GSIQN | IRSNERE | AWDSWGKII | SGHDY | FNHGAL | ASQLGSNEQY |
| 60 | GSIQN | IRSNERE | ATDAFGSVI | SGHDY | FNHGAL | ASQLGSNEQY |
| 61 | GSIQN | IRSNERE | ATDANGTVQ | SGHDY | FNHGAL | ASQLGSNEQY |
| 62 | GSIQN | IRSNERE | AYDQWGKII | SGHDY | FNHGAL | ASQLGPNEQY |
| 63 | GSIQN | IRSNERE | AWDSWGKII | SGHDY | FNHGAL | ASQLGPNEQY |
| 64 | GSIQN | IRSNERE | ATDAFGSVI | SGHDY | FNHGAL | ASQLGPNEQY |
| 65 | GSIQN | IRSNERE | ATDANGTVQ | SGHDY | FNHGAL | ASQLGPNEQY |
| 66 | QTPQN | IRSNERE | ATDANGKII | SGHDY | FNNNVP | ASSLGSNEQY |
| 67 | QTPQN | IRSNERE | ATDANGKII | SGHDY | FNHGAL | ASSLGSNEQY |
| 68 | QTPQN | IRSNERE | ATDANGKII | SGHDY | FNNNVP | ASQLGSNEQY |
| 69 | QTPQN | IRSNERE | ATDANGKII | SGHDY | FNNNVP | ASQLGPNEQY |
| 70 | QTPQN | IRSNERE | AYDQWGKII | SGHDY | FNHGAL | ASSLGSNEQY |
| 71 | QTPQN | IRSNERE | AWDSWGKII | SGHDY | FNHGAL | ASSLGSNEQY |
| 72 | QTPQN | IRSNERE | ATDAFGSVI | SGHDY | FNHGAL | ASSLGSNEQY |
| 73 | QTPQN | IRSNERE | ATDANGTVQ | SGHDY | FNHGAL | ASSLGSNEQY |
| 74 | QTPQN | IRSNERE | AYDQWGKII | SGHDY | FNHGAL | ASQLGSNEQY |
| 75 | QTPQN | IRSNERE | AWDSWGKII | SGHDY | FNHGAL | ASQLGSNEQY |
| 76 | QTPQN | IRSNERE | ATDAFGSVI | SGHDY | FNHGAL | ASQLGSNEQY |
| 77 | QTPQN | IRSNERE | ATDANGTVQ | SGHDY | FNHGAL | ASQLGSNEQY |
| 78 | QTPQN | IRSNERE | AYDQWGKII | SGHDY | FNHGAL | ASQLGPNEQY |
| 79 | QTPQN | IRSNERE | AWDSWGKII | SGHDY | FNHGAL | ASQLGPNEQY |
| 80 | QTPQN | IRSNERE | ATDAFGSVI | SGHDY | FNHGAL | ASQLGPNEQY |
| 81 | QTPQN | IRSNERE | ATDANGTVQ | SGHDY | FNHGAL | ASQLGPNEQY |
| 82 | VNPQN | IRSNERE | ATDANGKII | SGHDY | FNNNVP | ASSLGSNEQY |
| 83 | VNPQN | IRSNERE | ATDANGKII | SGHDY | FNHGAL | ASSLGSNEQY |
| 84 | VNPQN | IRSNERE | ATDANGKII | SGHDY | FNNNVP | ASQLGSNEQY |
| 85 | VNPQN | IRSNERE | ATDANGKII | SGHDY | FNNNVP | ASQLGPNEQY |
| 86 | VNPQN | IRSNERE | AYDQWGKII | SGHDY | FNHGAL | ASSLGSNEQY |
| 87 | VNPQN | IRSNERE | AWDSWGKII | SGHDY | FNHGAL | ASSLGSNEQY |
| 88 | VNPQN | IRSNERE | ATDAFGSVI | SGHDY | FNHGAL | ASSLGSNEQY |
| 89 | VNPQN | IRSNERE | ATDANGTVQ | SGHDY | FNHGAL | ASSLGSNEQY |
| 90 | VNPQN | IRSNERE | AYDQWGKII | SGHDY | FNHGAL | ASQLGSNEQY |
| 91 | VNPQN | IRSNERE | AWDSWGKII | SGHDY | FNHGAL | ASQLGSNEQY |
| 92 | VNPQN | IRSNERE | ATDAFGSVI | SGHDY | FNHGAL | ASQLGSNEQY |
| 93 | VNPQN | IRSNERE | ATDANGTVQ | SGHDY | FNHGAL | ASQLGSNEQY |
| 94 | VNPQN | IRSNERE | AYDQWGKII | SGHDY | FNHGAL | ASQLGPNEQY |
| 95 | VNPQN | IRSNERE | AWDSWGKII | SGHDY | FNHGAL | ASQLGPNEQY |
| 96 | VNPQN | IRSNERE | ATDAFGSVI | SGHDY | FNHGAL | ASQLGPNEQY |
| 97 | VNPQN | IRSNERE | ATDANGTVQ | SGHDY | FNHGAL | ASQLGPNEQY |

10. The TCR of claim 1, wherein the TCR is soluble.

11. The TCR of claim 1, wherein the TCR is an αβ heterodimeric TCR; preferably, the TCR has an α chain constant region sequence TRAC^{∗}01 and a β chain constant region sequence TRBC1^{∗}01 or TRBC2^{∗}01.

12. The TCR of claim 1, wherein the TCR comprises (i) all or part of a TCR α chain other than its transmembrane domain and (ii) all or part of a TCR β chain other than its transmembrane domain, wherein both of (i) and (ii) comprise a variable domain and at least part of a constant domain of the TCR chain.

13. The TCR of claim 12, wherein an artificial interchain disulfide bond is contained between an α chain constant region and a β chain constant region of the TCR.

14. The TCR of claim 13, wherein one or more groups of amino acids selected from the following are substituted by cysteine residues forming the artificial interchain disulfide bond between the α chain constant region and the β chain constant region of the TCR:
Thr48 of TRAC^{∗}01 exon 1 and Ser57 of TRBC1^{∗}01 or TRBC2^{∗}01 exon 1;
Thr45 of TRAC^{∗}01 exon 1 and Ser77 of TRBC1^{∗}01 or TRBC2^{∗}01 exon 1;
Tyr10 of TRAC^{∗}01 exon 1 and Ser17 of TRBC1^{∗}01 or TRBC2^{∗}01 exon 1;
Thr45 of TRAC^{∗}01 exon 1 and Asp59 of TRBC1^{∗}01 or TRBC2^{∗}01 exon 1;
Ser15 of TRAC^{∗}01 exon 1 and Glu15 of TRBC1^{∗}01 or TRBC2^{∗}01 exon 1;
Arg53 of TRAC^{∗}01 exon 1 and Ser54 of TRBC1^{∗}01 or TRBC2^{∗}01 exon 1;
Pro89 of TRAC^{∗}01 exon 1 and Ala19 of TRBC1^{∗}01 or TRBC2^{∗}01 exon 1; and
Tyr10 of TRAC^{∗}01 exon 1 and Glu20 of TRBC1^{∗}01 or TRBC2^{∗}01 exon 1.

15. The TCR of claim 1, wherein the amino acid sequence of the α chain variable domain of the TCR is selected from the group consisting of: SEQ ID NOs: 56-107; and/or the amino acid sequence of the β chain variable domain of the TCR is selected from the group consisting of: SEQ ID NOs: 108-122.

16. The TCR of claim 1, wherein the TCR is selected from the group consisting of:
| TCR No. | Sequence of a chain variable domain SEQ ID NO: | Sequence of β chain variable domain SEQ ID NO: |
|---|---|---|
| 27 | 82 | 2 |
| 22 | 77 | 2 |
| 1 | 56 | 2 |
| 2 | 57 | 2 |
| 3 | 58 | 2 |
| 4 | 59 | 2 |
| 5 | 60 | 2 |
| 6 | 61 | 2 |
| 7 | 62 | 2 |
| 8 | 63 | 2 |
| 9 | 64 | 2 |
| 10 | 65 | 2 |
| 11 | 66 | 2 |
| 12 | 67 | 2 |
| 13 | 68 | 2 |
| 14 | 69 | 2 |
| 15 | 70 | 2 |
| 16 | 71 | 2 |
| 17 | 72 | 2 |
| 18 | 73 | 2 |
| 19 | 74 | 2 |
| 20 | 75 | 2 |
| 21 | 76 | 2 |
| 23 | 78 | 2 |
| 24 | 79 | 2 |
| 25 | 80 | 2 |
| 26 | 81 | 2 |
| 28 | 83 | 2 |
| 29 | 84 | 2 |
| 30 | 85 | 2 |
| 31 | 86 | 2 |
| 32 | 87 | 2 |
| 33 | 88 | 2 |
| 34 | 89 | 2 |
| 35 | 1 | 108 |
| 36 | 1 | 109 |
| 37 | 1 | 110 |
| 38 | 1 | 111 |
| 39 | 1 | 112 |
| 40 | 1 | 113 |
| 41 | 1 | 114 |
| 42 | 1 | 115 |
| 43 | 1 | 116 |
| 44 | 90 | 2 |
| 45 | 91 | 2 |
| 46 | 1 | 117 |
| 47 | 1 | 118 |
| 48 | 85 | 117 |
| 49 | 84 | 117 |
| 50 | 85 | 119 |
| 51 | 85 | 120 |
| 52 | 92 | 2 |
| 53 | 93 | 2 |
| 54 | 94 | 117 |
| 55 | 95 | 117 |
| 56 | 96 | 117 |
| 57 | 97 | 117 |
| 58 | 94 | 121 |
| 59 | 95 | 121 |
| 60 | 96 | 121 |
| 61 | 97 | 121 |
| 62 | 94 | 122 |
| 63 | 95 | 122 |
| 64 | 96 | 122 |
| 65 | 97 | 122 |
| 66 | 98 | 2 |
| 67 | 98 | 117 |
| 68 | 98 | 119 |
| 69 | 98 | 120 |
| 70 | 99 | 117 |
| 71 | 100 | 117 |
| 72 | 101 | 117 |
| 73 | 102 | 117 |
| 74 | 99 | 121 |
| 75 | 100 | 121 |
| 76 | 101 | 121 |
| 77 | 102 | 121 |
| 78 | 99 | 122 |
| 79 | 100 | 122 |
| 80 | 101 | 122 |
| 81 | 102 | 122 |
| 82 | 103 | 2 |
| 83 | 103 | 117 |
| 84 | 103 | 119 |
| 85 | 103 | 120 |
| 86 | 104 | 117 |
| 87 | 105 | 117 |
| 88 | 106 | 117 |
| 89 | 107 | 117 |
| 90 | 104 | 121 |
| 91 | 105 | 121 |
| 92 | 106 | 121 |
| 93 | 107 | 121 |
| 94 | 104 | 122 |
| 95 | 105 | 122 |
| 96 | 106 | 122 |
| 97 | 107 | 122 |

17. The TCR of claim 1, wherein the TCR is a single chain TCR.

18. The TCR of claim 1, wherein the TCR is a single chain TCR consisting of an α chain variable domain and a β chain variable domain, wherein the α chain variable domain and the β chain variable domain are linked by a flexible short peptide sequence (linker).

19. The TCR of claim 18, wherein a hydrophobic core of the α chain variable domain and/or the β chain variable domain of the TCR is mutated.

20. The TCR of claim 1, wherein the amino acid sequence of the α chain variable domain of the TCR is selected from the group consisting of: SEQ ID NOs: 9-42; and/or the amino acid sequence of the β chain variable domain of the TCR is selected from the group consisting of: SEQ ID NOs: 43-51.

21. The TCR of claim 1, wherein the TCR is selected from the group consisting of:
| TCR No. | Sequence of a chain variable domain SEQ ID NO: | Sequence of β chain variable domain SEQ ID NO: |
|---|---|---|
| s-27 | 35 | 4 |
| s-22 | 30 | 4 |
| s-1 | 9 | 4 |
| s-2 | 10 | 4 |
| s-3 | 11 | 4 |
| s-4 | 12 | 4 |
| s-5 | 13 | 4 |
| s-6 | 14 | 4 |
| s-7 | 15 | 4 |
| s-8 | 16 | 4 |
| s-9 | 17 | 4 |
| s-10 | 18 | 4 |
| s-11 | 19 | 4 |
| s-12 | 20 | 4 |
| s-13 | 21 | 4 |
| s-14 | 22 | 4 |
| s-15 | 23 | 4 |
| s-16 | 24 | 4 |
| s-17 | 25 | 4 |
| s-18 | 26 | 4 |
| s-19 | 27 | 4 |
| s-20 | 28 | 4 |
| s-21 | 29 | 4 |
| s-23 | 31 | 4 |
| s-24 | 32 | 4 |
| s-25 | 33 | 4 |
| s-26 | 34 | 4 |
| s-28 | 36 | 4 |
| s-29 | 37 | 4 |
| s-30 | 38 | 4 |
| s-31 | 39 | 4 |
| s-32 | 40 | 4 |
| s-33 | 41 | 4 |
| s-34 | 42 | 4 |
| s-35 | 3 | 43 |
| s-36 | 3 | 44 |
| s-37 | 3 | 45 |
| s-38 | 3 | 46 |
| s-39 | 3 | 47 |
| s-40 | 3 | 48 |
| s-41 | 3 | 49 |
| s-42 | 3 | 50 |
| s-43 | 3 | 51 |

22. The TCR of any one of claims 1 to 21, wherein a conjugate is bound to an α chain and/or a β chain of the TCR at C- or N-terminal.

23. The TCR of claim 22, wherein the conjugate that binds to the TCR is a detectable label, a therapeutic agent, a PK modified moiety or a combination thereof.

24. The TCR of claim 23, wherein the therapeutic agent that binds to the TCR is an anti-CD3 antibody linked to the α or β chain of the TCR at C- or N-terminal.

25. A multivalent T-cell receptor (TCR) complex, comprising at least two TCR molecules, wherein at least one TCR molecule is the TCR of any one of claims 1 to 24.

26. A nucleic acid molecule, comprising a nucleic acid sequence for encoding the TCR of any one of claims 1 to 24 or a complementary sequence thereof.

27. A vector containing the nucleic acid molecule of claim 26.

28. A host cell containing the vector of claim 27 or having the exogenous nucleic acid molecule of claim 26 integrated into a chromosome of the host cell.

29. An isolated cell expressing the TCR of any one of claims 1 to 24.

30. A pharmaceutical composition containing a pharmaceutically acceptable carrier and the TCR of any one of claims 1 to 24 or the TCR complex of claim 25 or the cell of claim 29.

31. A method for treating a disease, comprising administering the TCR of any one of claims 1 to 24 or the TCR complex of claim 25 or the cell of claim 29 or the pharmaceutical composition of claim 30 to a subject in need thereof; preferably, the disease is NY-ESO-1 positive tumor.

32. A use of the T-cell receptor (TCR) of any one of claims 1 to 24 or the TCR complex of claim 25 or the cell of claim 29 for preparation of a medicament for treating a tumor, preferably, the tumor is NY-ESO-1 positive tumor.

33. A method for preparing the T-cell receptor (TCR) of any one of claims 1 to 24, comprising the steps of:
(i) culturing the host cell of claim 28 to express the TCR of any one of claims 1 to 24; and
(ii) isolating or purifying the TCR.
